# EUROPEAN PATENT APPLICATION

(11) **EP 3 438 255 A1**
(43) Date of publication of application: **06.02.2019**
(21) Application number: 17306041.9
(22) Date of filing: 03.08.2017
(51) Int. Cl.: C12N 9/88

(54) **HADD, A NOVEL PROTEIN OF THE MYCOBACTERIAL FATTY ACID SYNTHASE TYPE II**

(71) Applicant: Centre National de la Recherche Scientifique CNRS, 75794 Paris Cedex 16 (FR); Université Toulouse III-Paul Sabatier, 31062 Toulouse Cedex 9 (FR)
(72) Inventor: BARDOU, Fabienne, 31400 Toulouse (FR); BOULON, Richard, 31400 Toulouse (FR); BOUSQUET, Marie-Pierre, 31270 Cugnaux (FR); DAFFE, Mamadou, 31520 Ramonville Saint Agne (FR); DUCOUX-PETIT, Manuelle, 31520 Raimonville Saint Agne (FR); EYNARD, Nathalie, 31850 Beaupuy (FR); GAVALDA, Sabine, 31520 Raimonville Saint Agne (FR); LEFEBVRE, Cyril, 31400 Toulouse (FR); QUEMARD, Annaïk, 31450 Montgiscard (FR); BURLET-SCHILTZ, Odile, 31650 Saint Orens de Gameville (FR)
(74) Representative: Grosset-Fournier, Chantal Catherine

(57) **Abstract**

The application to a protein, which has been termed HadD by the inventors. HadD is an enzyme, which is involved in the biosynthesis of the mycobacterial envelope. A *hadD* orthologue is present in Non-Tuberculous Mycobacteria (NTM), as well as in tuberculous mycobacteria of the *M. tuberculosis* complex and in *M. leprae.* The application also relates to products and means, which involve or derive from the HadD protein, such as ligands. The application also relates to applications of said HadD protein, or of said products or means, notably for the identification of anti-mycobacterial compounds or for the treatment of mycobacterial diseases, more particularly of a NTM infection, tuberculosis or leprosy.

SEQUENCE LISTING

<110>  CENTRE NATIONAL DE LA RECHERCHE SCIENTIFIQUE
       UNIVERSITE PAUL SABATIER TOULOUSE III

<120>  HadD, a novel protein of the mycobacterial fatty acid synthase
       type II

<130>  MYCO

<160>  50

<170>  PatentIn version 3.5

<210>  1
<211>  177
<212>  PRT
<213>  M. smegmatis: MSMEG_0948

<400>  1

Met Ser Ile Ala Ala Asn Ile Ile Gly Thr His Tyr Arg Tyr Pro Asp
1               5                   10                  15

Tyr Phe Glu Val Gly Arg Glu Lys Val Arg Glu Phe Ser Ala Ala Val
            20                  25                  30

Lys Asp Asp His Pro Ala His Phe Asp Glu Ala Ala Ala Lys Glu Cys
        35                  40                  45

Gly His Asp Asn Leu Ile Ala Pro Leu Thr Phe Leu Ala Val Ala Gly
    50                  55                  60

Arg Arg Val Gln Leu Asp Leu Phe Asp Lys Phe Asp Val Pro Ile Asn
65                  70                  75                  80

Leu Glu Arg Val Leu His Arg Asp Gln Lys Leu Ile Phe His Arg Pro
                85                  90                  95

Ile Val Val Gly Asp Lys Leu Trp Phe Asp Ser Tyr Leu Asp Ser Val
            100                 105                 110

Ile Glu Ser His Gly Thr Val Ile Cys Glu Ile Arg Ala Glu Val Thr
        115                 120                 125

Asp Asp Asp Gly Lys Pro Val Ala Thr Ser Ile Val Thr Met Leu Gly
    130                 135                 140

Glu Ala Glu Ile Asp Glu Ala Asp Glu Ile Ser Ser Gln Ile Ala Ala
145                 150                 155                 160

Ala Arg Asp Ala Ala Ile Ala Lys Met Val Ala Gly Gln Lys Ser Ser
                165                 170                 175

Ser

<210>  2
<211>  166
<212>  PRT
<213>  M. tuberculosis H37Rv: Rv0504c

<400>  2

Met Thr Val Pro Glu Glu Ala Gln Thr Leu Ile Gly Lys His Tyr Arg
1               5                   10                  15

Ala Pro Asp His Phe Leu Val Gly Arg Glu Lys Ile Arg Glu Phe Ala
            20                  25                  30

Val Ala Val Lys Asp Asp His Pro Thr His Tyr Ser Glu Pro Asp Ala
        35                  40                  45

Ala Ala Ala Gly Tyr Pro Ala Leu Val Ala Pro Leu Thr Phe Leu Ala
    50                  55                  60

Ile Ala Gly Arg Arg Val Gln Leu Glu Ile Phe Thr Lys Phe Asn Ile
65                  70                  75                  80

Pro Ile Asn Ile Ala Arg Val Phe His Arg Asp Gln Lys Phe Arg Phe
                85                  90                  95

His Arg Pro Ile Leu Ala Asn Asp Lys Leu Tyr Phe Asp Thr Tyr Leu
            100                 105                 110

Asp Ser Val Ile Glu Ser His Gly Thr Val Leu Ala Glu Ile Arg Ser
        115                 120                 125

Glu Val Thr Asp Ala Glu Gly Lys Pro Val Val Thr Ser Val Val Thr
    130                 135                 140

Met Leu Gly Glu Ala Ala His His Glu Ala Asp Ala Asp Ala Thr Val
145                 150                 155                 160

Ala Ala Ile Ala Ser Ile
                165

<210>  3
<211>  166
<212>  PRT
<213>  M. tuberculosis Erdman: ERDMAN_0553

<400>  3

Met Thr Val Pro Glu Glu Ala Gln Thr Leu Ile Gly Lys His Tyr Arg
1               5                   10                  15

Ala Pro Asp His Phe Leu Val Gly Arg Glu Lys Ile Arg Glu Phe Ala
            20                  25                  30

Val Ala Val Lys Asp Asp His Pro Thr His Tyr Ser Glu Pro Asp Ala
        35                  40                  45

Ala Ala Ala Gly Tyr Pro Ala Leu Val Ala Pro Leu Thr Phe Leu Ala
    50                  55                  60

Ile Ala Gly Arg Arg Val Gln Leu Glu Ile Phe Thr Lys Phe Asn Ile
65                  70                  75                  80

Pro Ile Asn Ile Ala Arg Val Phe His Arg Asp Gln Lys Phe Arg Phe
                85                  90                  95

His Arg Pro Ile Leu Ala Asn Asp Lys Leu Tyr Phe Asp Thr Tyr Leu
            100                 105                 110

Asp Ser Val Ile Glu Ser His Gly Thr Val Leu Ala Glu Ile Arg Ser
        115                 120                 125

Glu Val Thr Asp Ala Glu Gly Lys Pro Val Val Thr Ser Val Val Thr
    130                 135                 140

Met Leu Gly Glu Ala Ala His His Glu Ala Asp Ala Asp Ala Thr Val
145                 150                 155                 160

Ala Ala Ile Ala Ser Ile
                165

<210>  4
<211>  166
<212>  PRT
<213>  M. tuberculosis H37Ra: MRA_0512

<400>  4

Met Thr Val Pro Glu Glu Ala Gln Thr Leu Ile Gly Lys His Tyr Arg
1               5                   10                  15

Ala Pro Asp His Phe Leu Val Gly Arg Glu Lys Ile Arg Glu Phe Ala
            20                  25                  30

Val Ala Val Lys Asp Asp His Pro Thr His Tyr Ser Glu Pro Asp Ala
        35                  40                  45

Ala Ala Ala Gly Tyr Pro Ala Leu Val Ala Pro Leu Thr Phe Leu Ala
    50                  55                  60

Ile Ala Gly Arg Arg Val Gln Leu Glu Ile Leu Thr Lys Phe Asn Ile
65                  70                  75                  80

Pro Ile Asn Ile Ala Arg Val Phe His Arg Asp Gln Lys Phe Arg Phe
                85                  90                  95

His Arg Pro Ile Leu Ala Asn Asp Lys Leu Tyr Phe Asp Thr Tyr Leu
            100                 105                 110

Asp Ser Val Ile Glu Ser His Gly Thr Val Leu Ala Glu Ile Arg Ser
        115                 120                 125

Glu Val Thr Asp Ala Glu Gly Lys Pro Val Val Thr Ser Val Val Thr
    130                 135                 140

Met Leu Gly Glu Ala Ala His His Glu Ala Asp Ala Asp Ala Thr Val
145                 150                 155                 160

Ala Ala Ile Ala Ser Ile
                165

<210>  5
<211>  166
<212>  PRT
<213>  M. bovis BCG pasteur: BCG_0547c

<400>  5

Met Thr Val Pro Glu Glu Ala Gln Thr Leu Ile Gly Lys His Tyr Arg
1               5                   10                  15

Ala Pro Asp His Phe Leu Val Gly Arg Glu Lys Ile Arg Glu Phe Ala
            20                  25                  30

Val Ala Val Lys Asp Asp His Pro Thr His Tyr Ser Glu Pro Asp Ala
        35                  40                  45

Ala Ala Ala Gly Tyr Pro Ala Leu Val Ala Pro Leu Thr Phe Leu Ala
    50                  55                  60

Ile Ala Gly Arg Arg Val Gln Leu Glu Ile Phe Thr Lys Phe Asn Ile
65                  70                  75                  80

Pro Ile Asn Ile Ala Arg Val Phe His Arg Asp Gln Lys Phe Arg Phe
                85                  90                  95

His Arg Pro Ile Leu Ala Asn Asp Lys Leu Tyr Phe Asp Thr Tyr Leu
            100                 105                 110

Asp Ser Val Ile Glu Ser His Gly Thr Val Leu Ala Glu Ile Arg Ser
        115                 120                 125

Glu Val Thr Asp Ala Glu Gly Lys Pro Val Val Thr Ser Val Val Thr
    130                 135                 140

Met Leu Gly Glu Ala Ala His His Glu Ala Asp Ala Asp Ala Thr Val
145                 150                 155                 160

Ala Ala Ile Ala Ser Ile
                165

<210>  6
<211>  166
<212>  PRT
<213>  M. africanum: MAF_05110

<400>  6

Met Thr Val Pro Glu Glu Ala Gln Thr Leu Ile Gly Lys His Tyr Arg
1               5                   10                  15

Ala Pro Asp His Phe Leu Val Gly Arg Glu Lys Ile Arg Glu Phe Ala
            20                  25                  30

Val Ala Val Lys Asp Asp His Pro Thr His Tyr Ser Glu Pro Asp Ala
        35                  40                  45

Ala Ala Ala Gly Tyr Pro Ala Leu Val Ala Pro Leu Thr Phe Leu Ala
    50                  55                  60

Ile Ala Gly Arg Arg Val Gln Leu Glu Ile Phe Thr Lys Phe Asn Ile
65                  70                  75                  80

Pro Ile Asn Ile Ala Arg Val Phe His Arg Asp Gln Lys Phe Arg Phe
                85                  90                  95

His Arg Pro Ile Leu Ala Asn Asp Lys Leu Tyr Phe Asp Thr Tyr Leu
            100                 105                 110

Asp Ser Val Ile Glu Ser His Gly Thr Val Leu Ala Glu Ile Arg Ser
        115                 120                 125

Glu Val Thr Asp Ala Glu Gly Lys Pro Val Val Thr Ser Val Val Thr
    130                 135                 140

Met Leu Gly Glu Ala Ala His His Glu Ala Asp Ala Asp Ala Thr Val
145                 150                 155                 160

Ala Ala Ile Ala Ser Ile
                165

<210>  7
<211>  166
<212>  PRT
<213>  M. microti: RN08_0563

<400>  7

Met Thr Val Pro Glu Glu Ala Gln Thr Leu Ile Gly Lys His Tyr Arg
1               5                   10                  15

Ala Pro Asp His Phe Leu Val Gly Arg Glu Lys Ile Arg Glu Phe Ala
            20                  25                  30

Val Ala Val Lys Asp Asp His Pro Thr His Tyr Ser Glu Pro Asp Ala
        35                  40                  45

Ala Ala Ala Gly Tyr Pro Ala Leu Val Ala Pro Leu Thr Phe Leu Ala
    50                  55                  60

Ile Ala Gly Arg Arg Val Gln Leu Glu Ile Phe Thr Lys Phe Asn Ile
65                  70                  75                  80

Pro Ile Asn Ile Ala Arg Val Phe His Arg Asp Gln Lys Phe Arg Phe
                85                  90                  95

His Arg Pro Ile Leu Ala Asn Asp Lys Leu Tyr Phe Asp Thr Tyr Leu
            100                 105                 110

Asp Ser Val Ile Glu Ser His Gly Thr Val Leu Ala Glu Ile Arg Ser
        115                 120                 125

Glu Val Thr Asp Ala Glu Gly Lys Pro Val Val Thr Ser Val Val Thr
    130                 135                 140

Met Leu Gly Glu Ala Ala His His Glu Ala Asp Ala Asp Ala Thr Val
145                 150                 155                 160

Ala Ala Ile Ala Ser Ile
                165

<210>  8
<211>  166
<212>  PRT

<213>  M. canettii CIPT: MCAN_05071

<400>  8

Met Thr Val Pro Glu Glu Ala Gln Thr Leu Ile Gly Lys His Tyr Arg
1               5                   10                  15

Ala Pro Asp Tyr Phe Leu Val Gly Arg Glu Lys Ile Arg Glu Phe Ala
            20                  25                  30

Val Ala Val Lys Asp Asp His Pro Thr His Tyr Ser Glu Pro Asp Ala
        35                  40                  45

Ala Ala Ala Gly Tyr Pro Ala Leu Val Ala Pro Leu Thr Phe Leu Ala
    50                  55                  60

Ile Ala Gly Arg Arg Val Gln Leu Glu Ile Phe Thr Lys Phe Asn Ile
65                  70                  75                  80

Pro Ile Asn Ile Ala Arg Val Phe His Arg Asp Gln Lys Phe Arg Phe
                85                  90                  95

His Arg Pro Ile Leu Ala Asn Asp Lys Leu Tyr Phe Asp Thr Tyr Leu
            100                 105                 110

Asp Ser Val Ile Glu Ser His Gly Thr Val Leu Ala Glu Ile Arg Ser
        115                 120                 125

Glu Val Thr Asp Ala Glu Gly Lys Pro Val Val Thr Ser Val Val Thr
    130                 135                 140

Met Leu Gly Glu Ala Ala His His Glu Ala Asp Ala Asp Ala Thr Val
145                 150                 155                 160

Ala Ala Ile Ala Ser Ile
                165


<210>  9
<211>  166
<212>  PRT
<213>  M. leprae TN: ML2425

<400>  9

Met Thr Val Pro Leu Glu Ala Glu Gly Leu Ile Gly Lys His Tyr Arg
1               5                   10                  15

Gln Leu Asp His Phe Gln Val Gly Arg Glu Lys Ile Arg Glu Phe Ala
            20                  25                  30

Ile Ala Val Lys Asp Asp His Pro Thr His Tyr Asn Glu Thr Ala Ala
        35                  40                  45

Phe Glu Ala Gly Tyr Pro Ala Leu Val Ala Pro Leu Thr Phe Leu Ala
    50                  55                  60

Ile Ala Gly Arg Arg Val Gln Leu Glu Ile Phe Thr Lys Phe Asn Ile
65                  70                  75                  80

Pro Ile Asn Val Ala Arg Val Phe His Arg Asp Gln Lys Phe Arg Phe
                85                  90                  95

Tyr Arg Thr Ile Leu Ala Gln Asp Lys Leu Tyr Phe Asp Thr Tyr Leu
            100                 105                 110

Asp Ser Val Ile Glu Ser His Gly Thr Val Ile Ala Glu Val Arg Ser
        115                 120                 125

Glu Val Thr Asp Thr Glu Gly Lys Ala Val Val Thr Ser Ile Val Thr
    130                 135                 140

Met Leu Gly Glu Leu Ala Arg Gln Asp Ala Thr Ala Glu Glu Thr Val
145                 150                 155                 160

Ala Ala Ile Ala Ser Ile
                165

<210>  10
<211>  174
<212>  PRT
<213>  M. avium: MAV_4646

<400>  10

Met Thr Val Pro Lys Glu Ala Glu Gly Leu Ile Gly Ser His Tyr Arg
1               5                   10                  15

Ala Pro Asp Tyr Phe Glu Val Gly Arg Glu Lys Ile Arg Glu Phe Ala
            20                  25                  30

Leu Ala Val Lys Asp Asp His Pro Ala His Phe Asp Glu Ser Glu Ser
        35                  40                  45

Ala Ala Ala Gly Tyr Pro Asp Met Val Ala Pro Leu Thr Phe Leu Ala
    50                  55                  60

Ile Ala Gly Arg Arg Val Gln Leu Glu Ile Phe Thr Lys Phe Ser Ile
65                  70                  75                  80

Pro Ile Asn Ile Ala Arg Val Ile His Arg Asp Gln Lys Phe Lys Phe
                85                  90                  95

His Arg Pro Ile Leu Ala His Asp Arg Leu Tyr Phe Asp Thr Tyr Leu
            100                 105                 110

Asp Ser Val Ile Glu Ser His Gly Thr Val Leu Ala Glu Ile Arg Ser
        115                 120                 125

Glu Val Thr Asp Ala Asp Gly Lys Pro Ile Val Thr Ser Val Val Thr
    130                 135                 140

Met Leu Gly Glu Ala Ala His Gln Glu Ala Asp Ala Ala Ala Thr Ala
145                 150                 155                 160

Ala Ala Val Ala Ser Ile Ser Ala Gly Lys Leu Gly Ala Ile
                165                 170

<210> 11
<211> 166
<212> PRT
<213> M. marinum M: MMAR_0832

<400> 11

Met Thr Val Pro Pro Glu Ala Ala Gly Leu Ile Gly Lys His Tyr Gln
1               5                   10                  15

Ala Pro Asp Tyr Phe Val Val Gly Arg Glu Lys Ile Arg Glu Phe Ala
            20                  25                  30

Thr Ala Ile Gln Asp Glu His Pro Ser His Ser Asp Glu Ala Ala Ala
        35                  40                  45

Ala Glu Leu Gly Tyr Pro Glu Leu Val Ala Pro Leu Thr Phe Leu Ala
    50                  55                  60

Ile Ala Gly Arg Arg Val Gln Leu Glu Ile Phe Asn Lys Phe Asn Ile
65                  70                  75                  80

Pro Ile Asn Ile Ala Arg Val Phe His Arg Asp Gln Lys Phe Arg Phe
                85                  90                  95

Tyr Arg Pro Ile Val Thr Gly Asp Lys Leu Tyr Phe His Thr Tyr Leu
            100                 105                 110

Asp Ser Val Leu Glu Ser His Gly Thr Val Ile Ala Glu Ile Arg Ser
        115                 120                 125

Glu Val Thr Asp Asp Glu Gly Lys Pro Val Val Thr Ser Val Val Thr
    130                 135                 140

Met Leu Gly Glu Ala Ala His His Glu Ala Glu Ala Asp Ala Ala Val
145                 150                 155                 160

Ala Ala Ile Ala Ser Ile
                165

<210>  12
<211>  166
<212>  PRT
<213>  M. ulcerans Agy99: MUL_4576

<400>  12

Met Thr Val Pro Pro Glu Ala Ala Gly Leu Ile Gly Lys His Tyr Gln
1               5                   10                  15

Ala Pro Asp Tyr Phe Val Val Gly Arg Glu Lys Ile Arg Glu Phe Ala
            20                  25                  30

Thr Ala Ile Gln Asp Glu His Pro Ser His Ser Asp Glu Ala Ala Ala
        35                  40                  45

Thr Glu Leu Gly Tyr Pro Glu Leu Val Ala Pro Leu Thr Phe Leu Ala
    50                  55                  60

Ile Ala Gly Arg Arg Val Gln Leu Glu Ile Phe Asn Lys Phe Asn Ile
65                  70                  75                  80

Pro Ile Asn Ile Ala Arg Val Phe His Arg Asp Gln Lys Phe Arg Phe
                85                  90                  95

Tyr Arg Pro Ile Val Thr Gly Asp Lys Leu Tyr Phe His Thr Tyr Leu
            100                 105                 110

Asp Ser Val Leu Glu Ser His Gly Thr Val Ile Ala Glu Ile Arg Ser
        115                 120                 125

Glu Val Thr Asp Asp Glu Gly Lys Pro Val Val Thr Ser Val Val Thr
    130                 135                 140

Met Leu Gly Glu Ala Ala His His Glu Ala Glu Ala Asp Ala Ala Val
145                 150                 155                 160

Ala Ala Ile Ala Ser Ile
                165

<210>  13
<211>  166
<212>  PRT
<213>  M. kansasii ATCC 12478: MkanA1_21523

<400>  13

Met Thr Val Pro Gln Glu Ala Glu Gly Leu Ile Gly Lys Asn Tyr Arg
1               5                   10                  15

Ala Pro Asp Tyr Phe Glu Val Gly Arg Glu Lys Ile Arg Glu Phe Ala
            20                  25                  30

Ala Ala Val Asn Asp Glu His Pro Ala His Tyr Asp Asp Ala Ala Ala
        35                  40                  45

Ala Asp Ala Gly Tyr Pro Ala Leu Val Ala Pro Pro Thr Phe Leu Ala
    50                  55                  60

Val Ala Gly Arg Arg Val Gln Met Glu Ile Phe Thr Lys Phe Ser Ile
65                  70                  75                  80

Pro Ile Asn Ile Ala Arg Val Phe His Arg Asp Gln Lys Phe Arg Phe
                85                  90                  95

His Arg Pro Ile Leu Ala Gly Asp Lys Leu Phe Phe His Thr Tyr Leu
            100                 105                 110

Asp Ser Val Ile Glu Ser His Gly Thr Val Leu Ala Glu Ile Arg Ser
        115                 120                 125

Glu Val Thr Asp Ala Asp Gly Lys Pro Val Ala Thr Gly Val Val Thr
    130                 135                 140

Met Leu Gly Glu Ala Ala His His Glu Ala Glu Ala Asp Ala Thr Val
145                 150                 155                 160

Ala Ala Ile Ala Ala Ile
                165


<210>  14
<211>  176
<212>  PRT
<213>  M. abscessus subsp. abscessus

<400>  14

Met Gly Ile Ala Asp Asp Ile Ile Gly Thr His Phe Arg Tyr Pro Asp
1               5                   10                  15

Tyr Phe Glu Val Gly Arg Glu Lys Val Lys Glu Phe Ala Gln Ala Val

20                  25                  30

Gln Asp Glu His Pro Ala His Phe Ser Glu Glu Ala Ala Ala Glu Cys
        35                  40                  45

Gly Ser Asp Thr Leu Ile Ala Ser Leu Thr Phe Ile Ala Val Ala Gly
    50                  55                  60

Arg Arg Val Gln Leu Glu Leu Phe Asn Gln Phe Asp Val Pro Ile Asn
65                  70                  75                  80

Leu Glu Arg Val Leu His Arg Asp Gln Lys Leu Ile Phe His Arg Pro
                85                  90                  95

Ile Lys Val Gly Asp Arg Leu Trp Phe Asp Ser Tyr Leu Asp Ser Val
            100                 105                 110

Ile Glu Ser His Gly Thr Val Ile Ala Glu Ile Arg Ala Glu Val Thr
        115                 120                 125

Asp Asp Ser Gly Glu Pro Ile Met Thr Ser Ile Val Thr Met Leu Gly
    130                 135                 140

Glu Ala Gln Asn Asp Asp Glu Ala Gly Glu Val Ser Ala Gln Ile Ala
145                 150                 155                 160

Ala Ala Arg Asp Ala Ala Ile Ala Lys Met Val Ala Ala Gln Lys Gly
                165                 170                 175

<210>  15
<211>  155
<212>  PRT
<213>  M. chelonae: BKG75_07330

<400>  15

Met Ala Ile Arg Gln Asp Ile Val Gly Thr His Tyr Arg Tyr Pro Asp
1               5                   10                  15

Tyr Phe Glu Val Gly Arg Glu Lys Met Arg Glu Phe Ala Ala Ala Ile
            20                  25                  30

Lys Asp Glu Cys Glu Val Asn Ser Gly Val Val Ala Pro Ile Thr Phe
        35                  40                  45

Leu Ala Val Ala Gly Arg Arg Val Gln His Lys Ile Phe Thr Glu Met
    50                  55                  60

Asp Leu Pro Ile Asn Val Ala Arg Val Leu His Arg Asp Gln Lys Leu

65                  70                  75                  80

Lys Phe His Arg Pro Ile Lys Ala Gly Asp Arg Leu Tyr Phe Asp Ser
                85                  90                  95

Tyr Leu Asp Ser Val Leu Glu Ser His Gly Met Val Thr Ala Glu Val
            100                 105                 110

Arg Ala Glu Val Thr Asp Ala Asp Gly Glu Pro Val Ala Thr Ser Ile
        115                 120                 125

Val Thr Met Ile Gly Glu Ala Glu Gly His Glu Thr Asp Ala Ala Ser
    130                 135                 140

Met Ala Ala Asn Ile Ala Ser His Arg Lys Ala
145                 150                 155

<210>  16
<211>  177
<212>  PRT
<213>  M. fortuitum : A5665_10040

<400>  16

Met Ser Ile Ala Ala Asn Ile Ile Gly Thr His Tyr Arg Tyr Pro Asp
1               5                   10                  15

Tyr Phe Glu Val Gly Arg Glu Lys Val Arg Glu Phe Ala Arg Ala Val
            20                  25                  30

Lys Asp Asp His Pro Ala Leu Tyr Asp Val Glu Ser Ala Lys Glu Phe
        35                  40                  45

Gly His Asp Ala Val Val Ala Pro Val Thr Phe Leu Ala Val Ala Gly
    50                  55                  60

Arg Arg Val Gln Leu Glu Leu Phe Asp Lys Phe Asp Ile Pro Ile Asn
65                  70                  75                  80

Leu Glu Arg Val Leu His Arg Asp Gln Lys Leu Ile Phe His Arg Pro
                85                  90                  95

Ile Met Val Gly Asp Lys Leu Trp Phe Asp Ser Tyr Leu Asp Ser Val
            100                 105                 110

Ile Glu Ser His Gly Thr Val Ile Ala Glu Val Arg Ala Glu Val Thr
        115                 120                 125

Asp Asp Asp Gly Asn Pro Val Ala Thr Ser Ile Val Thr Met Leu Gly

130                 135                 140

Glu Ala Ala Ile Asp Glu Ala Asp Glu Ile Ser Ser Gln Ile Ala Ala
145                 150                 155                 160

Ala Arg Asp Ala Ala Ile Ala Lys Met Val Ala Gly Gln Lys Ser Gly
                165                 170                 175

Ala

<210>  17
<211>  534
<212>  DNA
<213>  M. smegmatis: MSMEG_0948

<400>  17
atgagcatcg cggcaaacat catcgggact cactaccgct accccgacta tttcgaggtc      60

ggacgggaga aggtccgcga gttctcggcg gccgtcaagg acgaccaccc cgcgcatttc     120

gacgaggcgg cggccaagga gtgcggccac gacaacctca tcgcgccgct gaccttcctg     180

gcggtcgcgg gccgtcgagt gcagctcgac ctgttcgaca agttcgacgt gccgatcaac     240

ctcgagcgtg tgctgcaccg ggaccagaag ctgatcttcc accgtccgat cgtggtcggt     300

gacaagctgt ggttcgactc gtacctggac tcggtgatcg agtcgcacgg cacggtgatc     360

tgcgagatcc gtgccgaggt gaccgacgac gacggcaagc ccgtggccac cagcatcgtc     420

acgatgctcg gcgaggccga gatcgacgag gccgacgaga tcagttccca gatcgccgcg     480

gcgcgcgatg ccgccatcgc caagatggtt gccgggcaaa agtccagttc ctga           534

<210>  18
<211>  501
<212>  DNA
<213>  M. tuberculosis H37Rv: Rv0504c

<400>  18
atgacagttc ccgaagaagc ccagacactg atcggcaagc attatcgggc gccggaccac      60

tttctggtgg ggcgcgagaa gatccgcgag ttcgcggtcg cggtcaaaga cgaccacccg     120

acccactaca gcgagccgga tgccgcagcg gccggttacc ccgcgctggt agccccgctg     180

accttcctgg cgatcgccgg ccggcgcgtg cagctggaga tcttcaccaa gttcaatatc     240

ccgatcaaca tcgcccgggt ctttcatcgc gaccagaagt tccgcttcca ccggccgatc     300

ctggccaacg acaagctgta cttcgacacc tatcttgact ccgtcatcga atcccacggc     360

acggtgctcg ccgagatccg aagcgaagtt accgacgccg aaggaaagcc tgtggtcacc     420

agtgtcgtca ccatgttggg agaagcggcc caccacgaag ccgatgccga tgcaacggtg     480

gcggcgattg catcgatcta g                                               501

<210>  19
<211>  501
<212>  DNA
<213>  M. tuberculosis Erdman: ERDMAN_0553

<400>  19
atgacagttc ccgaagaagc ccagacactg atcggcaagc attatcgggc gccggaccac      60

tttctggtgg ggcgcgagaa gatccgcgag ttcgcggtcg cggtcaaaga cgaccacccg     120

acccactaca gcgagccgga tgccgcagcg gccggttacc ccgcgctggt agccccgctg     180

accttcctgg cgatcgccgg ccggcgcgtg cagctggaga tcttcaccaa gttcaatatc     240

ccgatcaaca tcgcccgggt ctttcatcgc gaccagaagt tccgcttcca ccggccgatc     300

ctggccaacg acaagctgta cttcgacacc tatcttgact ccgtcatcga atcccacggc     360

acggtgctcg ccgagatccg aagcgaagtt accgacgccg aaggaaagcc tgtggtcacc     420

agtgtcgtca ccatgttggg agaagcggcc caccacgaag ccgatgccga tgcaacggtg     480

gcggcgattg catcgatcta g                                               501

<210>  20
<211>  501
<212>  DNA
<213>  M. tuberculosis H37Ra: MRA_0512

<400>  20
atgacagttc ccgaagaagc ccagacactg atcggcaagc attatcgggc gccggaccac      60

tttctggtgg ggcgcgagaa gatccgcgag ttcgcggtcg cggtcaaaga cgaccacccg     120

acccactaca gcgagccgga tgccgcagcg gccggttacc ccgcgctggt agccccgctg     180

accttcctgg cgatcgccgg ccggcgcgtg cagctggaga tcttcaccaa gttcaatatc     240

ccgatcaaca tcgcccgggt cgttcatcgc gaccagaagt tccgcttcca ccggccgatc     300

ctggccaacg acaagctgta cttcgacacc tatcttgact ccgtcatcga atcccacggc     360

acggtgctcg ccgagatccg aagcgaagtt accgacgccg aaggaaagcc tgtggtcacc     420

agtgtcgtca ccatgttggg agaagcggcc caccacgaag ccgatgccga tgcaacggtg     480

gcggcgattg catcgatcta g                                               501

<210>  21
<211>  501
<212>  DNA
<213>  M. bovis BCG pasteur: BCG_0547c

<400>  21
atgacagttc ccgaagaagc ccagacactg atcggcaagc attatcgggc gccggaccac      60

tttctggtgg ggcgcgagaa gatccgcgag ttcgcggtcg cggtcaaaga cgaccacccg     120

acccactaca gcgagccgga tgccgcagcg gccggttacc ccgcgctggt agccccgctg     180

accttcctgg cgatcgccgg ccggcgcgtg cagctggaga tcttcaccaa gttcaatatc 240

ccgatcaaca tcgcccgggt ctttcatcgc gaccagaagt tccgcttcca ccggccgatc 300

ctggccaacg acaagctgta cttcgacacc tatcttgact ccgtcatcga atcccacggc 360

acggtgctcg ccgagatccg aagcgaagtt accgacgccg aaggaaagcc tgtggtcacc 420

agtgtcgtca ccatgttggg agaagcggcc caccacgaag ccgatgccga tgcaacggtg 480

gcggcgattg catcgatcta g 501

<210> 22
<211> 501
<212> DNA
<213> M. africanum: MAF_05110

<400> 22
atgacagttc ccgaagaagc ccagacactg atcggcaagc attatcgggc gccggaccac 60

tttctggtgg ggcgcgagaa gatccgcgag ttcgcggtcg cggtcaaaga cgaccacccg 120

acccactaca gcgagccgga tgccgcagcg gccggttacc ccgcgctggt agccccgctg 180

accttcctgg cgatcgccgg ccggcgcgtg cagctggaga tcttcaccaa gttcaatatc 240

ccgatcaaca tcgcccgggt ctttcatcgc gaccagaagt tccgcttcca ccggccgatc 300

ctggccaacg acaagctgta cttcgacacc tatcttgact ccgtcatcga atcccacggc 360

acggtgctcg ccgagatccg aagcgaagtt accgacgccg aaggaaagcc tgtggtcacc 420

agtgtcgtca ccatgttggg agaagcggcc caccacgaag ccgatgccga tgcaacggtg 480

gcggcgattg catcgatcta g 501

<210> 23
<211> 501
<212> DNA
<213> M. microti: RN08_0563

<400> 23
atgacagttc ccgaagaagc ccagacactg atcggcaagc attatcgggc gccggaccac 60

tttctggtgg ggcgcgagaa gatccgcgag ttcgcggtcg cggtcaaaga cgaccacccg 120

acccactaca gcgagccgga tgccgcagcg gccggttacc ccgcgctggt agccccgctg 180

accttcctgg cgatcgccgg ccggcgcgtg cagctggaga tcttcaccaa gttcaatatc 240

ccgatcaaca tcgcccgggt ctttcatcgc gaccagaagt tccgcttcca ccggccgatc 300

ctggccaacg acaagctgta cttcgacacc tatcttgact ccgtcatcga atcccacggc 360

acggtgctcg ccgagatccg aagcgaagtt accgacgccg aaggaaagcc tgtggtcacc 420

agtgtcgtca ccatgttggg agaagcggcc caccacgaag ccgatgccga tgcaacggtg 480

gcggcgattg catcgatcta g 501

<210>  24
<211>  501
<212>  DNA
<213>  M. canettii CIPT: MCAN_05071

<400>  24
atgacagttc ccgaagaagc ccagacactg atcggcaagc attatcgggc accggaccac      60

tttctggtgg ggcgcgagaa gatccgcgag ttcgcggtcg cggtcaaaga cgaccacccg     120

acccactaca gcgagccgga tgccgcagcg gccggttacc ccgcgctggt agccccgctg     180

accttcctgg cgatcgccgg ccggcgcgtg cagctggaga tcttcaccaa gttcaatatc     240

ccgatcaaca tcgcccgggt ctttcatcgc gaccagaagt tccgcttcca ccggccgatc     300

ctggccaacg acaagctgta cttcgacacc tatcttgact ccgtcatcga atcccacggc     360

acggtgctcg ccgagatccg aagcgaagtt accgacgccg aaggaaagcc tgtggtcacc     420

agtgtcgtca ccatgttggg agaagcggcc caccacgaag ccgatgccga tgcaacggtg     480

gcggcgattg catcgatcta g                                               501


<210>  25
<211>  501
<212>  DNA
<213>  M. leprae TN: ML2425

<400>  25
atgacagttc ccttagaggc cgaaggacta atcggcaagc attaccggca gttggatcac      60

ttccaagttg gacgcgagaa aatccgtgag ttcgcgatcg cggttaaaga cgaccaccca     120

acccactaca acgaaactgc agccttcgag gccggatacc ctgcactagt ggctccgcta     180

accttcctgg cgatcgctgg gcgacgcgtg cagctcgaga tttttactaa attcaacatc     240

ccgatcaatg tcgcccgggt tttccatcgc gaccaaaaat ttcgcttcta ccgcacgatc     300

ctggcacagg ataagctcta cttcgacacc tacctcgact cggtgatcga gtcacatggc     360

actgtgatcg cagaagtccg cagcgaggtc accgatacgg agggcaaggc ggtcgtcacc     420

agtattgtca ctatgctggg cgaattggcc cgtcaggatg ctaccgctga agaaaccgtc     480

gctgcaatcg cttctatatg a                                               501


<210>  26
<211>  525
<212>  DNA
<213>  M. avium: MAV_4646

<400>  26
atgacagtcc ccaaagaagc cgaaggactt atcggcagcc attaccgggc acccgactac      60

ttcgaggtcg gtcgcgagaa gatccgcgag ttcgcgctcg cggtcaaaga cgaccacccc     120

gcgcacttcg acgagagtga atccgccgca gctgggtacc ccgacatggt ggcgcccctc     180

acgttcctcg cgatcgcagg tcgccgcgtg cagctggaga tcttcaccaa gttcagcatc 240

cccatcaaca tcgccagggt catccaccgc gaccagaagt tcaagttcca tcgcccgatc 300

ctggcccacg acaggctgta tttcgacacc tatctagact cggtgatcga gtcccacggc 360

accgtgctcg cggagatccg cagcgaagtg accgatgccg atggaaagcc gattgtcacc 420

agcgtcgtca cgatgctggg ggaggccgca caccaagagg ccgacgcggc agcgaccgcc 480

gcggcagttg catccatatc agcaggaaag ttaggggcga tatag 525

<210> 27
<211> 501
<212> DNA
<213> M. marinum M: MMAR_0832

<400> 27
atgacagtcc ccccagaagc cgcaggactc atcggcaagc actaccaggc accggactac 60

ttcgtggtcg gccgcgagaa gatccgggaa ttcgcaaccg cgatccaaga cgagcaccca 120

tcgcactccg atgaggccgc cgcagccgag ttgggttacc ccgagctggt cgccccgttg 180

accttcctgg caattgcagg gcgtcgggtg cagctggaga tcttcaataa attcaatatt 240

ccgatcaaca tcgcccgggt cttccatcgg gaccagaagt tccgcttcta ccggcccatc 300

gtgaccggcg acaagctgta cttccacacc tatctcgact ctgtcctgga gtctcacggg 360

acggtgatag ccgaaatccg cagcgaggtc accgacgacg agggcaagcc ggtggtgacg 420

agtgtggtca cgatgttggg cgaggcggcc catcacgagg ccgaagcgga tgccgctgtg 480

gcggcgattg catccatcta g 501

<210> 28
<211> 501
<212> DNA
<213> M. ulcerans Agy99: MUL_4576

<400> 28
atgacagtcc ccccagaagc cgcaggactc atcggcaagc actaccaggc accggactac 60

ttcgtggtcg gccgcgagaa aatccgggaa ttcgcaaccg cgatccaaga cgagcaccca 120

tcgcactccg atgaggccgc cgcaaccgag ttgggttacc ccgagctggt cgccccgttg 180

accttcctgg caattgcagg gcgtcgggtg cagctggaga tcttcaataa attcaatatt 240

ccgatcaaca tcgcccgggt cttccatcgg gaccagaagt tccgcttcta ccggcccatc 300

gtgaccggcg acaagctgta cttccacacc tatctcgact ctgtcctgga gtctcacggg 360

acggtgatag ccgaaatccg cagcgaggtc accgacgacg agggcaagcc ggtggtgacg 420

agtgtggtca cgatgttggg cgaggcggcc catcacgagg ccgaagcgga tgccgctgtg 480

gcggcgattg catccatcta g 501

<210>  29
<211>  501
<212>  DNA
<213>  M. kansasii 1010001469: A4G31_17010

<400>  29
atgacagtcc cccaggaagc cgaaggactc atcggcaaga actaccgcgc cccggactac      60

ttcgaagtcg gccgggagaa gatccgcgag ttcgcggccg cggtcaacga cgagcatccg     120

gcgcactacg acgaggcggc cgcggccgac gcgggctacc ccgcattggt ggcgccgccg     180

accttcctgg cggtcgccgg gcggcgtgta cagatggaga ttttcaccaa gttcagcatt     240

ccgattaaca tcgcccgggt cttccatcgc gaccagaagt tccggttcca ccggccgatc     300

ctggccggcg acaaactttt ctttcatact tacctagact ctgtgattga gtcacacggc     360

acagtgctcg ccgagattcg aagtgaagtc acagatgccg acggcaagcc ggtcgctacc     420

ggcgtcgtca ccatgttggg ggaagcagcg caccacgagg ccgaggccga tgccaccgtg     480

gcggcaattg cagctatttg a                                               501

<210>  30
<211>  468
<212>  DNA
<213>  Mycobacterium abscessus

<400>  30
atggccatcc gccaagacat cgtcggaacg cactaccgct accccgatta cttcgaggtg      60

ggccgcgaga agatgcgcga attcgcggcg gccatcaagg acgagtgcga ggtcaactcc     120

ggcgtggtcg cgcccatcac cttcctggcg gtcgccggac gacgggtgca gcacaagata     180

ttcaccgaga tggacctacc catcaatgtc gcacgggtgc tgcaccgcga tcagaagctc     240

aagttccatc ggccgatccg ggtcggcgac cggctgtact tcgacagcta cctggacagc     300

gtcctggaat cgcacggcat ggtgaccgcc gaggtgcgcg ccgaggttac cgacgagaac     360

ggtgatcccg tcgccaccag catcgtgacc atgatcggcg aagccgaagg gcatgagcgg     420

gacgcggctg ccatggccgc gaacatcgcc tcacatcgca agaactag                  468

<210>  31
<211>  468
<212>  DNA
<213>  M. chelonae: BKG75_07330

<400>  31
atggccatcc gccaagacat cgtcggaacg cactaccgct accccgatta cttcgaggtc      60

ggccgcgaga agatgcgtga attcgccgcg gccatcaagg acgagtgcga ggtgaactcg     120

ggcgtcgtgg ctcccatcac cttcctggcg gtcgcgggcc ggcgggtgca gcacaagatc     180

ttcaccgaga tggatctgcc catcaacgtg gcacgcgtgt tgcaccgcga tcagaagctc     240

aagttccacc gccccatcaa ggcaggcgac cggctctact tcgatagcta cctggacagt     300

gtgctggagt cgcacggcat ggtgacggcc gaagtacggg ccgaggtgac cgatgccgac      360

ggtgaacccg tcgcgacgag catcgtgacc atgatcggcg aggccgaggg ccacgaaacc      420

gacgcggctt ccatggccgc gaacatcgcc tcacatcgca aggcttag                  468


<210>  32
<211>  534
<212>  DNA
<213>  M. fortuitum: A5665_10040

<400>  32
atgagcatcg ccgcaaacat catcgggact cactaccgct accccgacta cttcgaggtc       60

ggccgggaga aggtccgtga gttcgctcgc gcggtcaagg acgaccatcc cgcgctctac      120

gacgtcgagt cggccaagga gttcggtcac gacgcggtcg tggcgccggt gaccttcctg      180

gccgtggccg gccgtcgcgt ccagctcgag ctgttcgaca agttcgacat cccgatcaac      240

ctggagcgcg tgctgcaccg cgatcagaag ctgatcttcc accggcccat catggtcggc      300

gacaagctgt ggttcgactc gtatctggac tcggtcatcg aatcgcacgg caccgtgatc      360

gccgaggtgc gcgccgaggt caccgatgac gacggcaacc cggttgccac gagcatcgtc      420

accatgctgg gcgaggcggc catcgacgag gccgatgaga tcagttcaca gatcgccgcg      480

gcacgcgatg ccgcgatcgc caagatggtt gccgggcaaa agtccggcgc ctga            534


<210>  33
<211>  174
<212>  DNA
<213>  Artificial

<220>
<223>  sequence of knocked out M. tuberculosis Rv0504c gene

<400>  33
atgacagttc ccgaagaagc ccagacactg atcggcaagc ttgccgagat ccgaagcgaa       60

gttaccgacg ccgaaggaaa gcctgtggtc accagtgtcg tcaccatgtt gggagaagcg      120

gcccaccacg aagccgatgc cgatgcaacg gtggcggcga ttgcatcgat ctag            174


<210>  34
<211>  792
<212>  DNA
<213>  Artificial

<220>
<223>  streptomycin resistance cassette replacing the deleted M.
        smegmatis HadD gene

<400>  34
atgagggaag cggtgatcgc cgaagtatcg actcaactat cagaggtagt tggcgtcatc       60

gagcgccatc tcgaaccgac gttgctggcc gtacatttgt acggctccgc agtggatggc      120

ggcctgaagc cacacagtga tattgatttg ctggttacgg tgaccgtaag gcttgatgaa 180

acaacgcggc gagctttgat caacgacctt ttggaaactt cggcttcccc tggagagagc 240

gagattctcc gcgctgtaga agtcaccatt gttgtgcacg acgacatcat tccgtggcgt 300

tatccagcta agcgcgaact gcaatttgga gaatggcagc gcaatgacat tcttgcaggt 360

atcttcgagc cagccacgat cgacattgat ctggctatct tgctgacaaa agcaagagaa 420

catagcgttg ccttggtagg tccagcggcg gaggaactct ttgatccggt tcctgaacag 480

gatctatttg aggcgctaaa tgaaacctta acgctatgga actcgccgcc cgactgggct 540

ggcgatgagc gaaatgtagt gcttacgttg tcccgcattt ggtacagcgc agtaaccggc 600

aaaatcgcgc cgaaggatgt cgctgccgac tgggcaatgg agcgcctgcc ggcccagtat 660

cagcccgtca tacttgaagc tagacaggct tatcttggac aagaagaaga tcgcttggcc 720

tcgcgcgcag atcagttgga agaatttgtc cactacgtga aaggcgagat caccaaggta 780

gtcggcaaat aa 792

<210> 35
<211> 28
<212> PRT
<213> Artificial

<220>
<223> Degenerate hydratase 2 motif of M. smegmatis HadB


<220>
<221> MISC_FEATURE
<222> (3)..(3)
<223> X is any aa

<220>
<221> MISC_FEATURE
<222> (4)..(4)
<223> X is any aa

<220>
<221> MISC_FEATURE
<222> (15)..(15)
<223> X is any aa

<220>
<221> MISC_FEATURE
<222> (22)..(22)
<223> X is any aa

<220>
<221> MISC_FEATURE
<222> (23)..(23)
<223> X is any aa

<220>
<221> MISC_FEATURE
<222> (24)..(24)
<223> X is any aa

<220>
<221>  MISC_FEATURE
<222>  (25)..(25)
<223>  X is any aa

<220>
<221>  MISC_FEATURE
<222>  (26)..(26)
<223>  X is any aa

<400>  35

Tyr Phe Xaa Xaa Leu Ile Val Gly Ser Thr Gly Cys Gly Asp Xaa Asn
1               5                   10                  15

Pro Leu Ile Val His Xaa Xaa Xaa Xaa Xaa Ala Ser
            20                  25

<210>  36
<211>  27
<212>  PRT
<213>  Artificial

<220>
<223>  Degenerate hydratase 2 motif of M. smegmatis HadB

<220>
<221>  MISC_FEATURE
<222>  (2)..(2)
<223>  X is any aa

<220>
<221>  misc_feature
<222>  (3)..(3)
<223>  Xaa can be any naturally occurring amino acid

<220>
<221>  MISC_FEATURE
<222>  (14)..(14)
<223>  X is any aa

<220>
<221>  MISC_FEATURE
<222>  (21)..(21)
<223>  X is any aa

<220>
<221>  MISC_FEATURE
<222>  (22)..(22)
<223>  X is any aa

<220>
<221>  MISC_FEATURE
<222>  (23)..(23)
<223>  X is any aa

<220>
<221>  MISC_FEATURE
<222>  (24)..(24)

<223>  X is any aa

<220>
<221>  MISC_FEATURE
<222>  (25)..(25)
<223>  X is any aa

<400>  36

Tyr Phe Xaa Leu Ile Val Gly Ser Thr Gly Cys Gly Asp Xaa Asn Pro
1               5                   10                  15

Leu Ile Val His Xaa Xaa Xaa Xaa Xaa Ala Ser
            20                  25

<210>  37
<211>  18
<212>  PRT
<213>  Artificial

<220>
<223>  Degenerate hydratase 2 motif of M. smegmatis HadB

<220>
<221>  MISC_FEATURE
<222>  (2)..(2)
<223>  X is any aa

<220>
<221>  MISC_FEATURE
<222>  (3)..(3)
<223>  X is any aa

<220>
<221>  MISC_FEATURE
<222>  (5)..(5)
<223>  X is any aa

<220>
<221>  MISC_FEATURE
<222>  (6)..(6)
<223>  X is any aa

<220>
<221>  MISC_FEATURE
<222>  (8)..(8)
<223>  X is any aa

<220>
<221>  MISC_FEATURE
<222>  (9)..(9)
<223>  X is any aa

<220>
<221>  MISC_FEATURE
<222>  (13)..(13)
<223>  X is any aa

<220>
<221>  MISC_FEATURE

<222>  (14)..(14)
<223>  X is any aa

<220>
<221>  MISC_FEATURE
<222>  (15)..(15)
<223>  X is any aa

<220>
<221>  MISC_FEATURE
<222>  (16)..(16)
<223>  X is any aa

<220>
<221>  MISC_FEATURE
<222>  (17)..(17)
<223>  X is any aa

<400>  37

Phe Xaa Xaa Ala Xaa Xaa Asp Xaa Xaa Pro Ala His Xaa Xaa Xaa Xaa
1               5                   10                  15

Xaa Ala

<210>  38
<211>  18
<212>  PRT
<213>  Mycobacterium smegmatis

<400>  38

Phe Ser Ala Ala Val Lys Asp Asp His Pro Ala His Phe Asp Glu Ala
1               5                   10                  15

Ala Ala

<210>  39
<211>  8
<212>  PRT
<213>  Artificial

<220>
<223>  Generic version of HadD degenerate hydratase motif

<220>
<221>  MISC_FEATURE
<222>  (2)..(2)
<223>  X is F or Y

<220>
<221>  MISC_FEATURE
<222>  (3)..(3)
<223>  X is A or S

<220>

<221>  MISC_FEATURE
<222>  (4)..(4)
<223>  X is any aa

<220>
<221>  MISC_FEATURE
<222>  (5)..(5)
<223>  X is A or S

<220>
<221>  MISC_FEATURE
<222>  (6)..(6)
<223>  X is V or I

<220>
<221>  MISC_FEATURE
<222>  (7)..(7)
<223>  X is any aa

<220>
<221>  MISC_FEATURE
<222>  (7)..(7)
<223>  X is any aa, more particularly K, Q or N

<220>
<221>  MISC_FEATURE
<222>  (8)..(8)
<223>  X is D or N or S, more particularly D or N, more particularly D

<400>  39

Glu Xaa Xaa Xaa Xaa Xaa Xaa Xaa
1               5


<210>  40
<211>  8
<212>  PRT
<213>  Mycobacterium smegmatis

<400>  40

Glu Phe Ser Ala Ala Val Lys Asp
1               5


<210>  41
<211>  8
<212>  PRT
<213>  Mycobacterium tuberculosis

<400>  41

Glu Phe Ala Val Ala Val Lys Asp
1               5


<210>  42
<211>  8
<212>  PRT
<213>  Mycobacterium leprae

<400>  42

Glu Phe Ala Ile Ala Val Lys Asp
1               5

<210>  43
<211>  8
<212>  PRT
<213>  Mycobacterium avium

<400>  43

Glu Phe Ala Leu Ala Val Lys Asp
1               5

<210>  44
<211>  8
<212>  PRT
<213>  Mycobacterium marinum

<400>  44

Glu Phe Ala Thr Ala Ile Gln Asp
1               5

<210>  45
<211>  8
<212>  PRT
<213>  Mycobacterium kansasii

<400>  45

Glu Phe Ala Ala Ala Val Asn Asp
1               5

<210>  46
<211>  8
<212>  PRT
<213>  Mycobacterium abscessus

<400>  46

Glu Phe Ala Gln Ala Val Gln Asp
1               5

<210>  47
<211>  8
<212>  PRT
<213>  Mycobacterium chelonae

<400>  47

Glu Phe Ala Ala Ala Ile Lys Asp
1               5

<210>  48
<211>  8
<212>  PRT

<213>  Mycobacterium fortuitum

<400>  48

Glu Phe Ala Arg Ala Val Lys Asp
1               5


<210>  49
<211>  19
<212>  PRT
<213>  Artificial

<220>
<223>  Particular example of HadD degenerate hydratase motif


<220>
<221>  MISC_FEATURE
<222>  (3)..(3)
<223>  X is any aa

<220>
<221>  MISC_FEATURE
<222>  (4)..(4)
<223>  X is any aa

<220>
<221>  MISC_FEATURE
<222>  (6)..(6)
<223>  X is any aa

<220>
<221>  MISC_FEATURE
<222>  (7)..(7)
<223>  X is any aa

<220>
<221>  MISC_FEATURE
<222>  (9)..(9)
<223>  X is any aa

<220>
<221>  MISC_FEATURE
<222>  (10)..(10)
<223>  X is any aa

<220>
<221>  MISC_FEATURE
<222>  (14)..(14)
<223>  X is any aa

<220>
<221>  MISC_FEATURE
<222>  (15)..(15)
<223>  X is any aa

<220>
<221>  MISC_FEATURE
<222>  (16)..(16)
<223>  X is any aa

<220>

<221>  MISC_FEATURE
<222>  (17)..(17)
<223>  X is any aa

<220>
<221>  MISC_FEATURE
<222>  (18)..(18)
<223>  X is any aa

<400>  49

Glu Phe Xaa Xaa Ala Xaa Xaa Asp Xaa Xaa Pro Ala His Xaa Xaa Xaa
1               5                   10                  15

Xaa Xaa Ala

<210>  50
<211>  19
<212>  PRT
<213>  Mycobacterium smegmatis

<400>  50

Glu Phe Ser Ala Ala Val Lys Asp Asp His Pro Ala His Phe Asp Glu
1               5                   10                  15

Ala Ala Ala

## Description

### FIELD

The application relates to a novel mycobacterial protein, which has been termed HadD by the inventors. HadD is an enzyme. It is involved in the biosynthesis of the mycobacterial envelope. The *hadD* gene is present in Non-Tuberculous Mycobacteria (NTM), as well as in tuberculous mycobacteria of the *M. tuberculosis* complex and in *M. leprae. HadD* is however absent from other genera of the *Corynebacteriales* order.

The application also relates to products and means, which involve or derive from the HadD protein, such as ligands or nucleic acids, and to applications thereof, notably in the fields of the identification of anti-mycobacterial compounds or of the treatment of mycobacterial diseases, more particularly the treatment of a NTM infection, of tuberculosis or of leprosy.

### BACKGROUND

Mycobacteria include pathogens known to cause serious diseases in mammals, such as tuberculosis (*e.g., Mycobacterium tuberculosis*) and leprosy (*e.g., Mycobacterium leprae*) in humans. Mycobacteria also include Non-Tuberculous Mycobacteria (NTM). Tuberculosis is a global health threat, infecting about one third of the human population. Antimycobacterial therapy exists, but is only partially effective because of the propensity of mycobacteria of the tuberculosis complex (such as *M. tuberculosis*) to develop resistance. One additional problem is that mycobacteria of the *M. tuberculosis* complex often have the capacity to persist within infected hosts for a long period of time.

The nontuberculous mycobacteria (NTM), such as *M. smegmatis, M. avium, M. marinum, M. ulcerans, M. kansasii, M. abscessus, M. chelonae, M. fortuitum, M. xenopi, M. simiae* and *M. szulgai,* form a heterogeneous group of environmental organisms found in water, soil and dust. Pathogenic NTM can cause infectious diseases in livestock and wildlife, as well as human diseases, such as pulmonary and disseminated infections, lymphadenitis, and skin and soft tissue infections. They constitute the major cause of opportunistic infections in HIV patients, but infect immuno-competent individuals as well. Alarmingly, the number of reported cases worldwide has been increasing since the 1950s (Soni, De Groote et al. 2016; Nishiuchi, Iwamoto et al. 2017). NTM also colonize man-made environments and cause healthcare-associated infections, which are a major public health concern. NTM organized in biofilms are difficult to eradicate with common decontamination practices and are relatively resistant to standard disinfectants and to high concentrations of antimicrobial drugs (Sousa, Bandeira et al. 2015). Furthermore, many of the anti-tuberculosis drugs under development are inactive against NTM. Thus, novel therapeutic strategies for eradicating NTM from infection sources and hosts are urgently needed (Soni, De Groote et al. 2016). The very thick envelope of mycobacteria is characterized by a broad array of exotic complex lipids, such as the mycolate-containing compounds, which are key players in the infectious processes of pathogenic species. The mycolate-containing compounds are made of mycolic acids (MAs), unusually long α-alkylated β-hydroxylated fatty acids, esterifying either the extremities of the polysaccharide arabinogalactan or polyol molecules such as trehalose, forming the trehalose monomycolate (TMM) and the trehalose dimycolate (TDM) (Quemard 2016). The anchoring of the mycolate-containing lipids in the outer membrane (or mycomembrane) confers them a strategic position within the envelope, greatly impacting its architecture and its permeability. Large amounts of free MAs are also present in the extracellular matrix of mycobacterial biofilms (Ojha, Baughn et al. 2008; Ojha, Trivelli et al. 2010). The mycolate-containing compounds are essential for the survival of mycobacteria and crucial for their physiology and fitness (Quemard 2016). Furthermore, they modulate the immune response to infection by pathogenic mycobacteria, thereby playing a role in their virulence and their persistence within the host (Verschoor, Baird et al. 2012; Slama, Jamet et al. 2016). MAs are the products of a mixed fatty acid synthase (FAS)/polyketide synthase biosynthesis pathway (Quemard 2016), which represents a validated source of pharmacological targets (Marrakchi, Laneelle et al. 2014; North, Jackson et al. 2014). In particular, their main very long 'meromycolic' chain is produced by the FAS type II (FAS-II) system via iterative elongation cycles. MA subclasses typifying the different mycobacterial species are characterized by various types of chemical function (cyclopropane ring, double bond, methyl branch and oxygenated functions), at the proximal and distal positions of their main 'meromycolic' chain (Marrakci, Laneelle et al. 2014), which modulate the biological properties of the molecules (Verschoor, Baird et al. 2012). The enzymatic differentiation of the meromycolic chains is achieved by a family of paralogous S-adenosylmethionine-dependent MA methyltransferases (MA-MTs) (Marrakchi, Laneelle et al. 2014), most likely using a mechanism of C-methylation of isolated *cis* double bonds via the formation of an intermediate carbonium ion, demonstrated in the case of the *trans*-ethylenic MAs *of M. smegmatis* (Lederer 1969). While most steps of the MA biosynthesis pathway are well characterized (Quemard 2016), the mechanisms by which the double bonds are introduced in their meromycolic chain are still under investigation.

### SUMMARY

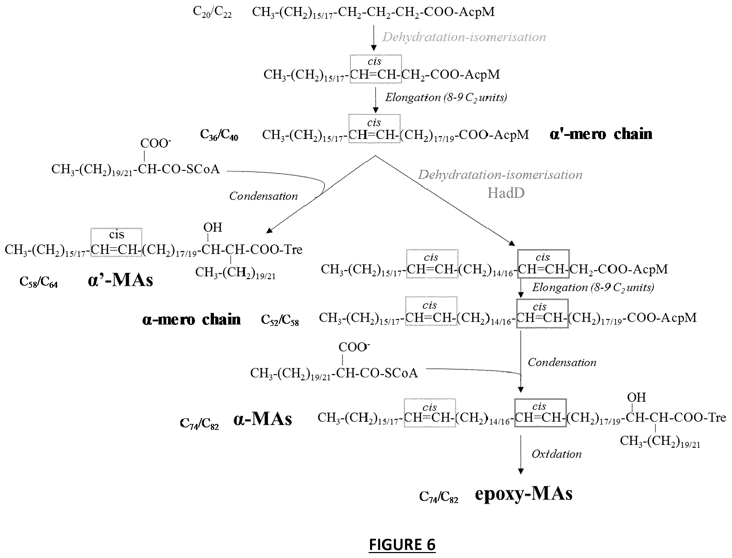

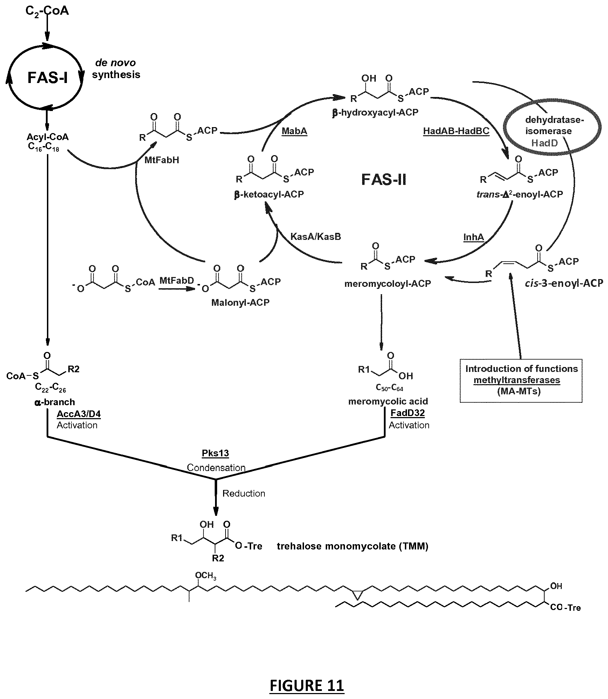

The application to a protein, which has been termed HadD by the inventors. HadD is an enzyme, more particularly a *Mycobacterium* enzyme. HadD may be viewed as a dehydratase-like (and optionally as an isomerase-like) enzyme of the mycobacterial fatty acid synthase type II (*cf.* Figures 6 and 11).

HadD is involved in the biosynthesis of the mycobacterial envelope and more particularly in the biosynthesis of mycolic acids of the mycobacterial envelope.

The corresponding *hadD* gene is found in Non-Tuberculous Mycobacteria (NTM), such as *Mycobacterium smegmatis, M. avium, M. marinum, M. ulcerans, M. kansasii, M. abscessus, M. chelonae, M. fortuitum, M. xenopi, M. simiae* and *M. szulgai,* as well as in mycobacteria of the *M. tuberculosis* complex (*M. tuberculosis, M. bovis, M. africanum, M. microti, M. canettii, M. caprae, M. orygis, M. mungi and M. pinnipedii*) and in *M. leprae.* No *hadD* orthologue is however present in other genera of the *Corynebacteriales* order, such as *Corynebacteria, Nocardia, Rhodococcus* and *Gordonia.*

The application also relates to products and means, which involve or derive from the HadD protein, such as ligands, protein complexes, protein crystals, nucleic acids, nucleic acid vectors, genetically engineered cells, genetically modified *Mycobacterium* cells (more particularly an attenuated *Mycobacterium* cell).

The application also relates to applications of said HadD protein, or of said products or **m**eans, notably in the field of the identification of anti-mycobacterial compounds and in the treatment of mycobacterial diseases, more particularly of a NTM infection, tuberculosis or leprosy.

### LEGEND

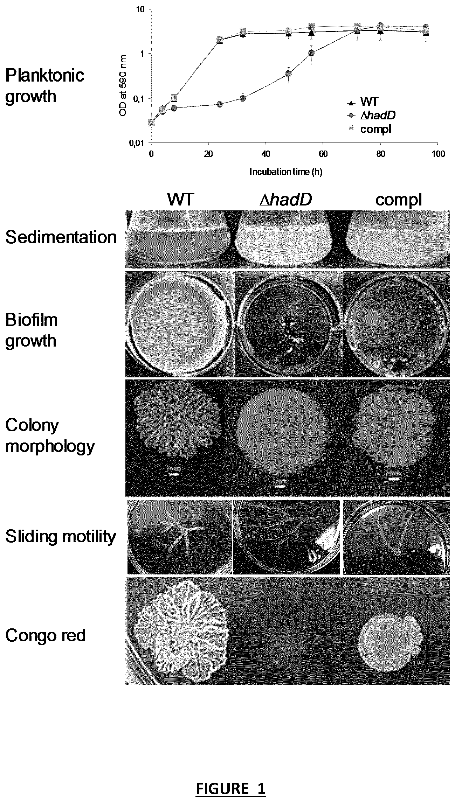

**Figure 1****.** *MSMEG*_*0948* (*i.e., hadD*) knock-out in *M. smegmatis* results in reduced growth in planktonic cultures, slower sedimentation, impaired biofilm growth, drastic change in colony morphology, increased sliding motility and intense Congo red staining.

Comparaison of *M. smegmatis* wt, *ΔhadD* and complemented (compl) strains in different phenotyping assays. All the pictures are representative of at least three independent experiments. Growth curves were determined at 37°C in Middlebrook 7H9-based medium supplemented with 0.05% (w/v) TWEEN-80. Data are means and standard deviations of three independent experiments. Sedimentation assays: cultures in 7H9-based medium with 0.05% (w/v) TWEEN-80 were allowed to decant for 15 min. Biofilm growth: it was monitored in Sauton's medium after 6 and 10 days of incubation at 37°C. These photographs correspond to a 6-day incubation; similar differences were observed after 10 days. Colony morphology: 5 µl culture aliquots were spotted on 7H10-based medium. Sliding motility: it was monitored after 7-day incubation on semi-solid medium. Congo red: 5 µl preculture aliquots were spotted on 7H10-based medium supplemented with 100 µg/ml Congo red.

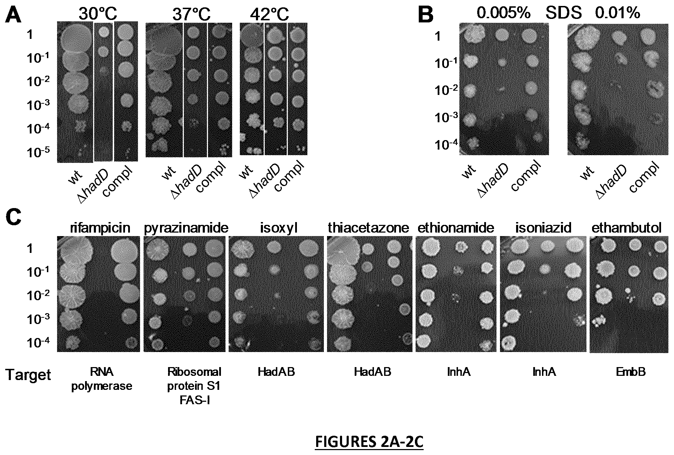

**Figures 2A-2C****.** *MSMEG*_*0948* (*i.e., hadD*) knock-out in *M. smegmatis* results in higher sensitivity to low temperature (30°C) and to the detergent SDS, as well as increased sensitivity to antituberculosis drugs.

Comparaison of *M. smegmatis* wt, *ΔhadD* and complemented (compl) strains in different assays. Cultures were serially diluted then spotted on Middlebrook 7H10 medium supplemented with the required drug or detergent and incubated at 37°C in the standard conditions or at 30°C and 42°C for temperature testing. All the photographs are representative of at least three independent experiments. (2A) Susceptibility to temperature. (2B) Susceptibility to SDS. (2C) Susceptibility to TB drugs. The target protein of each drug is mentioned.

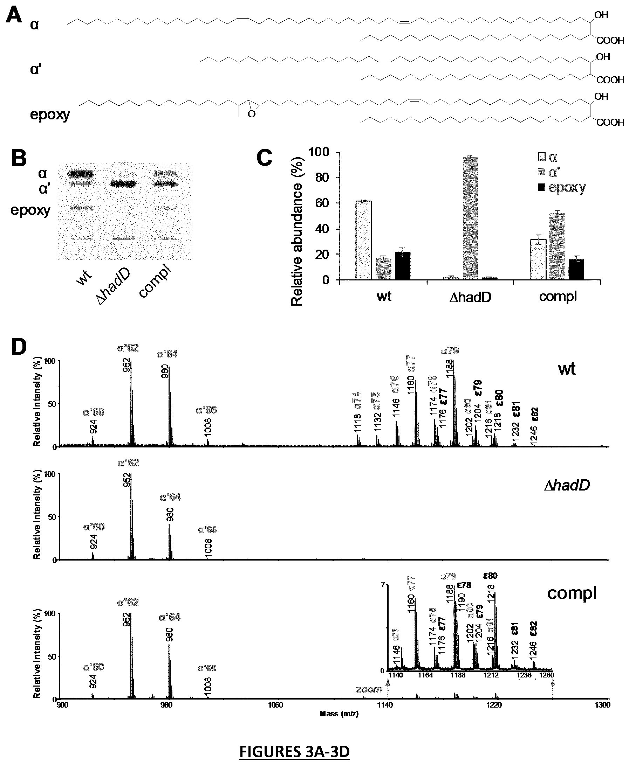

**Figures 3A-3D****.** Mycolic acids produced by *M. smegmatis.* Alpha, alpha' and epoxy mycolic acids (3A). *MSMEG*_*0948 (i.e., hadD*) knock-out in *M. smegmatis* results in a drastic decrease in cell-wall-linked alpha and epoxy mycolic acids, and in a relative increase in alpha' mycolic acids (3B-3D).

(3A) Structures of the main MAs from *M. smegmatis.* (3B) HPTLC analysis of MAME profiles in the wt, *ΔhadD* and complemented (compl) strains. Five µg of each MA mixture were loaded onto a HPTLC plate developed in petroleum ether/diethyl ether (9:1, v/v), and stained with CuSO4. (3C) MA distribution in each strain deduced from the quantification of the HPTLC bands in panel (3B) by measurement at 400 nm. Data are means and standard deviations of three independent experiments. (3D) MALDI-TOF MS spectra of the total MAME mixtures. The major ion peaks (corresponding to sodium adducts) are labelled with the matching MA type and the total carbon number (of the free acid form). ε, epoxy-MAs. The spectra are representative of four independent experiments.

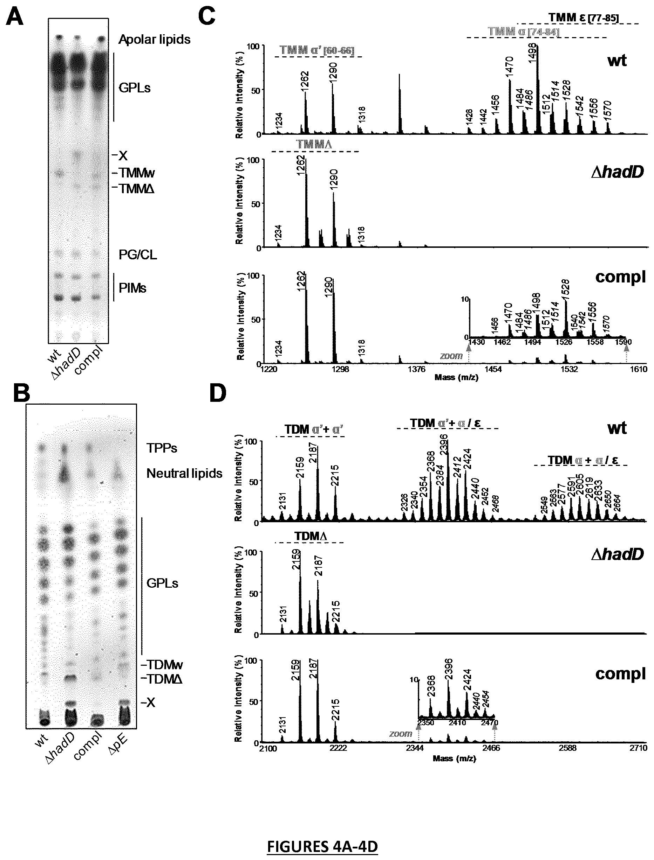

**Figures 4A-4D****.** Analysis of the extractable mycolate-containing lipids of *M. smegmatis ΔMSMEG_0948* (*i*.*e*., *ΔhadD*)*.* MALDI-TOF (4C-4D). TMM = trehalose monomycolate. TDM = trehalose dimycolate. *MSMEG*_*0948* (*i.e., hadD*) knock-out in *M. smegmatis* results in the production of TMM and TDM homologs bearing only α'-mycoloyl chains, which display a smaller *R*f in thin layer chromatography.

(4A) and (4B) TLC analyses of the total extractable lipids. 100 µg of each lipid mixture were loaded on TLC plates developed either in CHCl₃:CH₃OH:H₂O (65:25:4, v/v/v) (in panel A) or in CHCl₃:CH₃OH (9:1, v/v) (in panel B). The spots were revealed by anthrone spraying and heating. CL, cardiolipids; GPLs, glycopeptidolipids; PIMs, phosphatidylinositol mannosides; PG, phosphatidyl glycerol; TDMw, trehalose dimycolate mixture of the wt strain; TDMΔ, TDMs of the mutant strain; TMMw, trehalose monomycolate mixture of the wt strain; TMMΔ, TMMs of the mutant strain; TPPs, trehalose polyphleates. Compl, complemented strain; Δ*pE*, pE acyltranferase deficient mutant. (4C) and (4D) MALDI-TOF MS spectra of purified TMMs and TDMs, respectively. The major ion peaks (corresponding to sodium adducts) are labelled with the mycoloyl chain composition and total carbon number. ε, epoxy-MAs.

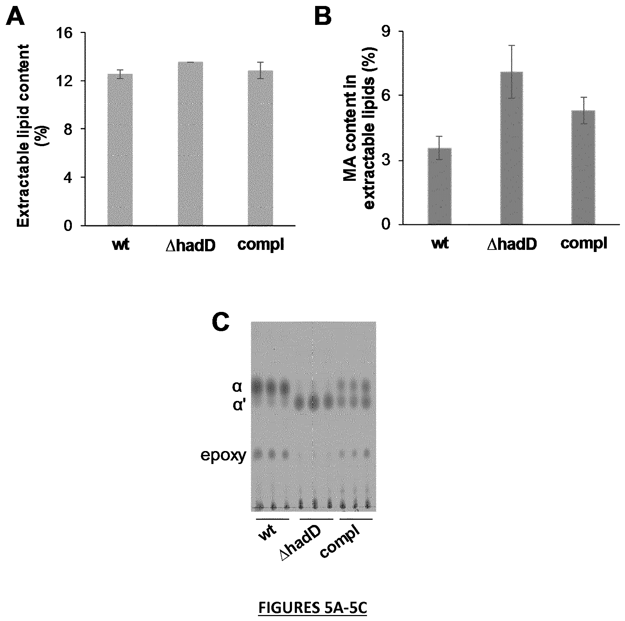

**Figures 5A-5C****.** Analysis of the extractable lipids of *M*. *smegmatis ΔMSMEG_0948* (*i.e., ΔhadD*)*.*

(5A) Total extractable lipid (TEL) content in the *M. smegmatis* wt, *ΔhadD* and complemented (compl) strains. Values expressed as percentages were calculated as follows: 100 * TEL dry weight / (TEL dry weight+delipidated residue dry weight). (5B) Total MA content in the extractable lipids. Values expressed as percentages were calculated as follows: 100 * dry weight of MAs from TEL / TEL dry weight. (5C) TLC analysis of the mycolic acyl chains from the extractable lipids (TMMs and TDMs). Equivalent amounts of mycolic acid methyl ester (MAME) mixtures from independent triplicates were spotted onto a thin layer developed in dichlolomethane and revealed by CuSO₄ spraying and heating.

**Figure 6****.** Proposed mechanistic contribution of HadD: synthesis of the second (proximal) *cis*-double bond in the meromycolic chain, which is required for the subsequent formation of the third hydrocarbon segment present in alpha- and epoxy-MAs, but absent from alpha'-MAs. MA = mycolic acid.

The dehydration/isomerization of a C₂₀-C₂₂ fatty acyl-AcpM intermediate allows the introduction of the future distal *cis* double bond in the meromycolic chain. After a first set of FAS-II elongation cycles, there is a Claisen-type condensation of the resulting short α'-meromycoloyl chains with a carboxy-C₂₂/C₂₄ fatty acyl-CoA by Pks13 enzyme, generating the α'-MAs. A second dehydration/isomerization of the α'-meromycoloyl (mero) chains by HadD introduces the future proximal *cis* double bond. After a second set of FAS-II elongation cycles, there is a condensation of the resulting long α-meromycoloyl chains with a carboxy-C₂₂/C₂₄ fatty acyl-CoA, generating the α-MAs. Finally, an oxidation reaction allows the biosynthesis of the epoxy-MAs. The total carbon number of the main *cis* ethylenic (mero)mycolic chains are mentioned.

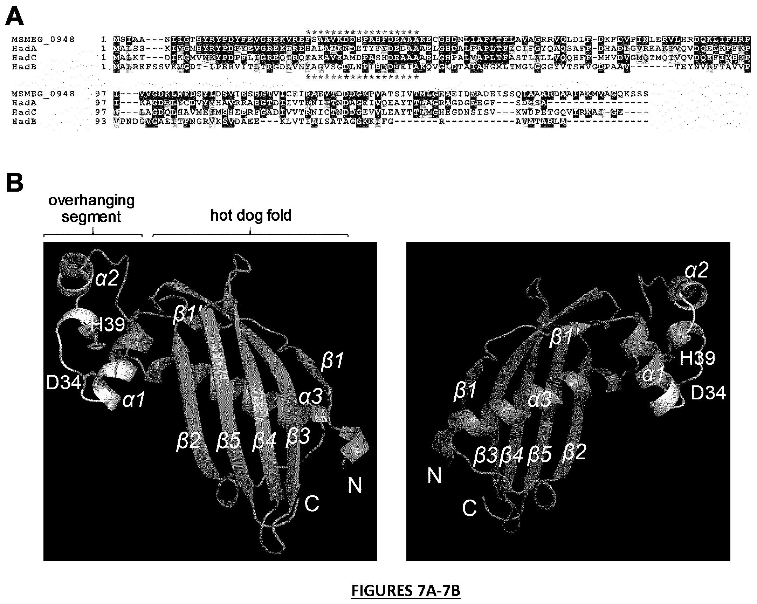

**Figures 7A-7B****.** Structural analysis of MSMEG_0948 (i.e., HadD) protein.

(7A) Sequence alignment with HadA, HadC and HadB from *M. smegmatis.* Black and gray shadings indicate strictly conserved and similar residues, respectively. MSMEG_0948 shares a sequence identity of 41% with HadA, 35% with HadC, and only 21% with HadB (CLUSTAL OMEGA scores). The HadA/HadC and HadA/HadB identity rates are 46% and 19%, respectively. MSMEG_0948 bears a degenerate hydratase 2 motif 'F-x(2)-a-x(2)-D-x(2)-P-x-H-x(5)-A' (SEQ ID NO: 37) indicated above the sequence by stars; the putative catalytic Asp and His residues are labeled by black stars. The hydratase 2 motif '[YF]-x(1,2)-[LIVG]-[STGC]-G-D-x-N-P-[LIV]-H-x(5)-[AS]' of HadB (SEQ ID NO: 35; SEQ ID NO: 36) is indicated below the sequence by stars. Alignment was performed by using CLUSTAL OMEGA program, and the figure was shaped with BOXSHADE. Database accession numbers: MSMEG_0948, A0QR13 (177 aa); HadA, A0QS40 (158 aa); HadB, A0QS41 (142 aa); HadC, A0QS42 (169 aa).

(7B) Homologous modelling. Ribbon representation of MSMEG_0948 monomeric model. MSMEG_0948 would organize, like HadA and HadC, as a single 'hot dog' fold made of a central α-helix wrapped into a five-stranded antiparallel β-sheet. The degenerate hydratase 2 motif (residues 28-45) is colored in white. The secondary structure elements are labelled according to HadA structure (Dong, Qiu et al. 2015). Figure performed using PYMOL software (http://www.pymol.org).

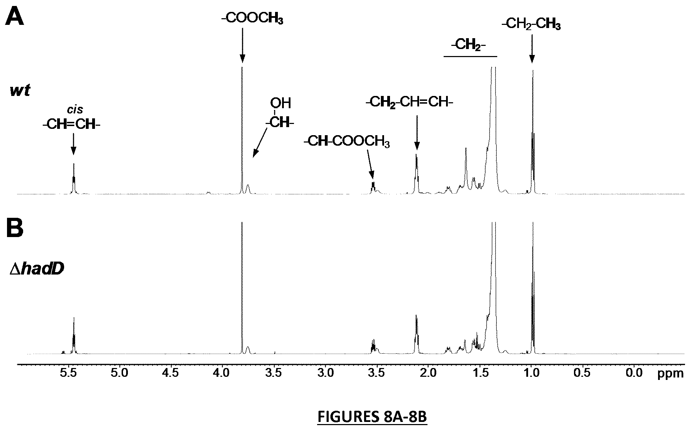

**Figures 8A-8B****.** ¹H -NMR analysis of the α'-mycolic acids from *M. smegmatis ΔMSMEG_0948* and wt strains. Both spectra are superimposable. ¹H-NMR spectra of purified α'-MAs from *M. smegmatis* wt strain (A) and *ΔhadD* strain (B). The proton(s) generating the signals are in bold.

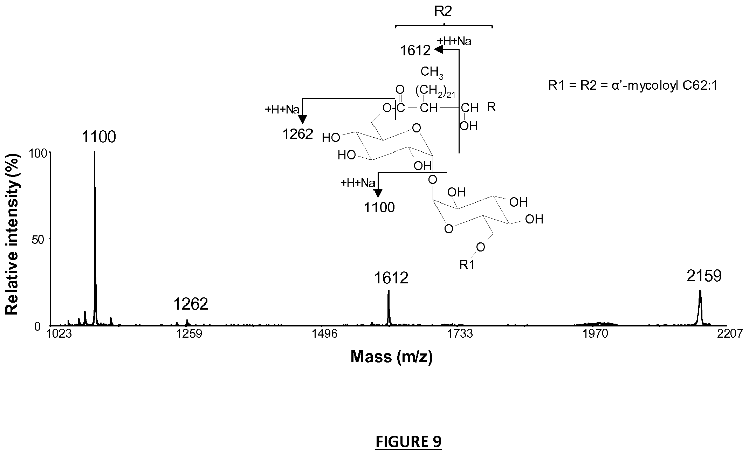

**Figure 9****.** MALDI-TOF/TOF tandem MS analysis of TDMΔ. TDM = trehalose dimycolate. Example of fragmentation of the TDM from *M. smegmatis ΔhadD* (TDMΔ) of *m*/*z* 2159.

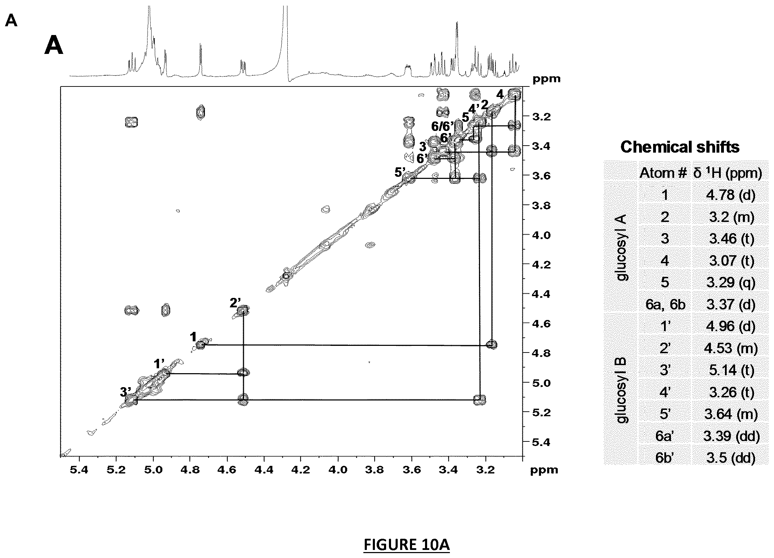

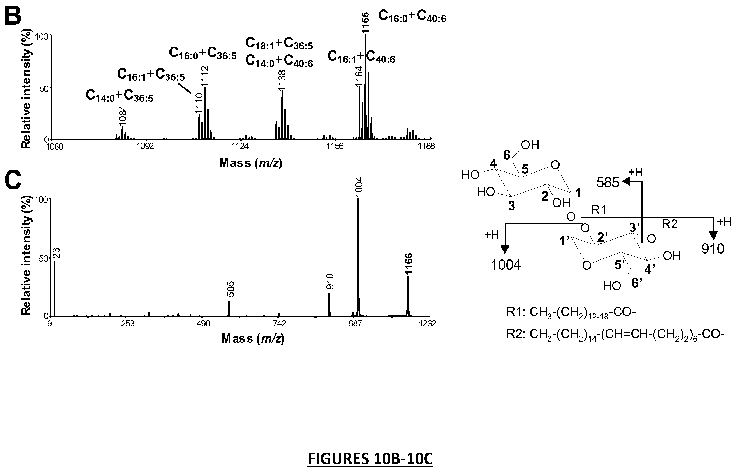

**Figures 10A**-**10C**. **Structural analyses of purified compound X.** These analyses show that compound X corresponds to a mixture of 2-fatty acyl,3-phleate trehalose homologs. (A) Magnification of the ¹H-¹H-COSY NMR spectrum. The spin systems of the glucosyl A and the glucosyl B esterified by two fatty acids are indicated. The numbers refer to the protons of the glucosyl residues as defined in the table and in the structure in panel (C). Chemical shifts are indicated in the table; multiplicities are annotated in parentheses: d, doublet; dd, doublet of doublet; t, triplet; m, multiplet. (B) MALDI-TOF MS spectrum. The major ion peaks (corresponding to sodium adducts) are labelled with their fatty acyl composition (R1+R2). (C) and MALDI-TOF/TOF MS/MS spectrum. Example of fragmentation of the ion of *m*/*z* 1166 (see panel (B)).

**Figure 11****.** Mycolic acid biosynthesis pathway. It involves FAS-I and FAS-II systems as well as the mycolic condensation system. The proposed reaction step catalyzed by HadD during FAS-II elongation cycles is displayed.

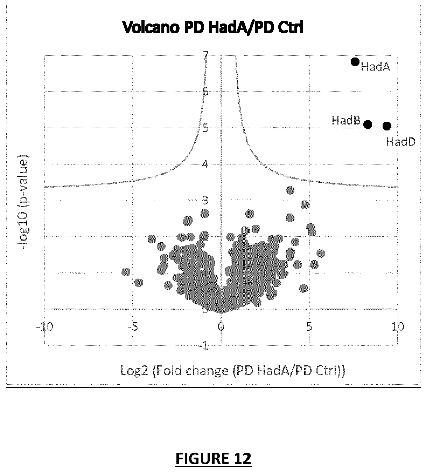

**Figure 12****.** Proteomics analysis of pull-down fractions using eGFP-HadA as a bait. Volcano plot [-log10(p-value) versus log2(fold change)] showing enriched proteins in eGFP-HadA-based pull-down (PD) versus control pull-down (using simple eGFP as a bait). Statistical analysis was performed from four independent biological replicates by two-sided Student t-test, variance correction and permutation-based false discovery rate (FDR) control in Perseus. Proteins considered as significantly enriched (FDR < 5%, hyperbolic selection curve indicated in grey) are colored in black.

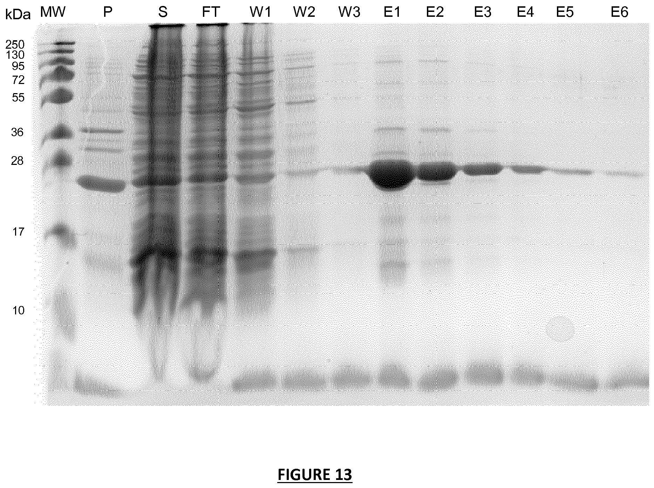

**Figure 13****.** SDS-PAGE of purification of MSMEG_0948 (HadD). N-terminal polyHis-tagged HadD (H-HadD) was produced in *E. coli rosetta* (DE3) after induction by IPTG. After lysis, the soluble protein mixture (S) was loaded onto Cobalt TALON ® Superflow Metal Affinity Resin. After wash cycles (W1 to W3 fractions), H-HadD (21.7 kDa) was eluted using 200 mM imidazole (E1 to E4 elution fractions). MW: molecular weight standards, P: pellet, S: total soluble protein mixture, FT: flow through, W: wash fractions, E: elution fractions.

### DETAILED DESCRIPTION

The inventors have discovered a new protein and termed it "HadD". The inventors have identified HadD from mycobacteria, and have characterized the structure and function of HadD, more particularly with respect to the biosynthesis of the mycobacterial envelope. The application relates to the HadD protein, as well as to means and applications that involve or derive from HadD.

The HadD protein advantageously is an enzyme, more particularly a mycobacterial enzyme. HadD may be viewed as a dehydratase-like (and optionally as an isomerase-like) enzyme of the mycobacterial fatty acid synthase type II (*cf.* Figures 6 and 11).

HadD is involved in the biosynthesis of the mycobacterial envelope, more particularly in the biosynthesis of mycolic acids of the mycobacterial envelope.

The corresponding *hadD* gene is found in
- Non-Tuberculous Mycobacteria (NTM), such as *Mycobacterium smegmatis, M. avium, M. marinum, M. ulcerans, M. kansasii, M. abscessus, M. chelonae, M. fortuitum, M. xenopi, M. simiae* and *M. szulgai,* as well as in
- mycobacteria of the *M. tuberculosis* complex (*M*. *tuberculosis, M. bovis, M. africanum, M. microti, M. canettii, M. caprae, M. orygis, M. mungi and M. pinnipedii*) and in
- *M. leprae.*

No *hadD* orthologue was found in other genera of the *Corynebacteriales* order, such as *Corynebacteria, Nocardia, Rhodococcus* and *Gordonia.*

The HadD protein can be defined as a protein, the amino acid sequence of which is
- the sequence of SEQ ID NO: 1, or
- an amino acid sequence, which is at least 59% identical to SEQ ID NO: 1 (over the entire length of SEQ ID NO: 1).

The expression "at least 59%" encompasses at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 87%, at least 88%, at least 89%, at least 90%, at least 91%, at least 92%, at least 93%, at least 94%, at least 95%, at least 96%, at least 97%, at least 98%, at least 99%.

Said protein may have retained the amino acid motif of SEQ ID NO: 39, more particularly an amino acid motif chosen from among SEQ ID NOs: 40-48.

The amino acid motif of SEQ ID NO: 39 is EXXXXXXX, wherein
X at position 2 is F or Y,
X at position 3 is A or S,
X at position 4 is any amino acid, more particularly A, V, I, L, T or R,
X at position 5 is A or S,
X at position 6 is V, I or L more particularly V or I
X at position 7 is any amino acid, more particularly K, Q or N, and
X at position 8 is D, N or S, more particularly D or N, more particularly D.

The amino acid motif of SEQ ID NO: 40 is EFSAAVKD, and may be found in the HadD sequence of e.g., *M. smegmatis.*

The amino acid motif of SEQ ID NO: 41 is EFAVAVKD, and may be found in the HadD sequence of e.g., *M. tuberculosis.*

The amino acid motif of SEQ ID NO: 42 is EFAIAVKD, and may be found in the HadD sequence of e.g., *M. leprae.*

The amino acid motif of SEQ ID NO: 43 is EFALAVKD, and may be found in the HadD sequence of e.g., *M. avium.*

The amino acid motif of SEQ ID NO: 44 is EFATAIQD, and may be found in the HadD sequence of e.g., *M. marinum or M. ulcerans.*

The amino acid motif of SEQ ID NO: 45 is EFAAAVND, and may be found in the HadD sequence of e.g., *M. kansasii.*

The amino acid motif of SEQ ID NO: 46 is EFAQAVQD, and may be found in the HadD sequence of e.g., *M. abscessus.*

The amino acid motif of SEQ ID NO: 47 is EFAAAIKD, and may be found in the HadD sequence of e.g., *M. chelonae.*

The amino acid motif of SEQ ID NO: 48 is EFARAVKD, and may be found in the HadD sequence of e.g., *M. fortuitum.*

Said protein may have retained the enzymatic activity of a dehydratase, more particularly the enzymatic activity of a dehydratase and the enzymatic activity of an isomerase.

The enzymatic activity of a dehydratase includes an (enzymatic) dehydration. The person of ordinary skill in the art understands that a dehydratase catalyzes a reversible reaction, i.e., a reaction that includes dehydration and hydration.

The amino acid motif of SEQ ID NO: 39, more particularly the amino acid motifs of SEQ ID NO: 40-48, may each be viewed as a dehydratase motif.

The protein may e.g., be in isolated or purified form.

Said protein may be obtainable by isolation (or purification) from a mycobacterium, more particularly from
- a Non-Tuberculous Mycobacterium (NTM), such as *Mycobacterium smegmatis, M. avium, M. marinum, M. ulcerans, M. kansasii, M. abscessus, M. chelonae, M. fortuitum, M. xenopi, M. simiae* and *M. szulgai*; more particularly from *Mycobacterium smegmatis, M. avium, M. marinum, M. ulcerans* or *M. kausasii;* or from
- a non-NTM mycobacterium, such as
   a mycobacterium of the tuberculosis complex (*M*. *tuberculosis, M. bovis, M. africanum, M. microti, M. canettii, M. caprae, M. orygis, M. mungi and M. pinnipedii*)*,* more particularly from *M. tuberculosis,* or such as
   *M. leprae.*

Said protein may be obtainable by pull-down experiment using at least one bait protein selected from the group consisting of the Fatty Acid Synthase type II (FAS-II) enzymes of mycobacterial cells, e.g., pull-down experiment wherein said mycobacterial cells are lysed under non-denaturing conditions and an affinity ligand (e.g., affinity purification beads) is contacted with said lysed cells or with a protein extract thereof to bind said at least one bait protein and to pull-down proteins that are bound to said at least one bait protein, and by isolation (or purification) from the pull-down fraction.

More particularly, said protein may be obtainable by
- lysing mycobacterial cells under non-denaturing conditions,
- contacting said lysed cells, or a protein extract thereof, with an affinity ligand or with an antibody (more particularly with an affinity ligand, such as e.g., affinity purification beads), wherein said affinity ligand or antibody (is covalently linked or) binds to at least one bait protein binds to at least one bait protein and which co-precipitates proteins that are bound to said at least one bait protein, wherein said at least one bait protein is selected from the group consisting of the Fatty Acid Synthase type II (FAS-II) enzymes of mycobacterial cells, and
- isolating or purifying the protein from the proteins, which co-precipitate with said at least one bait protein.

Contacting may be achieved e.g., by incubating (and/or by mixing).

Cell lysis in non-denaturing conditions is known to the person of average skill in the art. It may e.g., include collection of the cells, placing said cells in contact with a non-denaturing lysis buffer to lyse said cells, and collecting the cell lysate (e.g., by centrifugation of the lysed cells and collection of the supernatant).

Non-denaturing lysis buffers are known to the person of average skill in the art. A non-denaturing lysis buffer may e.g., contain one or several lysis detergents, wherein said lysis detergents are non-denaturing lysis detergents e.g., one or several lysis detergents chosen from among TRITON® X100, NP40, TWEEN 20, bile salts (such as cholate) and CHAPS (3-[(3-cholamidopropyl)dimethylammonio]-1-propanesulfonate).

Said mycobacterial cells may e.g., be:
- cells of a Non-Tuberculous Mycobacterium (NTM), such as *Mycobacterium smegmatis, M. avium, M. marinum, M. ulcerans, M. kansasii, M. abscessus, M. chelonae, M. fortuitum, M. xenopi, M. simiae* and *M. szulgai*; more particularly cells of *Mycobacterium smegmatis, M. avium, M. marinum, M. ulcerans* or *M. kansasii*; or
- cells of a non-NTM mycobacterium, such as a mycobacterium of the tuberculosis complex (*M*. *tuberculosis, M. bovis, M. africanum, M. microti, M. canettii, M. caprae, M. orygis, M. mungi and M. pinnipedii*)*,* more particularly *M. tuberculosis,* or such as *M. leprae.*

Said at least one bait protein may advantageously be selected from the group consisting of mycobacterial HadA, mycobacterial HadB, mycobacterial HadC, mycobacterial HadAB, mycobacterial HadBC, mycobacterial MabA, mycobacterial KasA, mycobacterial KasB, mycobacterial lnhA and mycobacterial AcpM, more particularly from the group consisting of mycobacterial HadA, mycobacterial HadB and mycobacterial HadC, more particularly from the group consisting of mycobacterial HadA, mycobacterial HadB, mycobacterial HadC, mycobacterial HadAB and mycobacterial HadBC, more particularly from the group consisting of mycobacterial HadA, mycobacterial HadB and mycobacterial HadC.

Advantageously, said protein may be not obtainable by isolation (or purification) from *Corynebacterium, Nocardia, Rhodococcus* and *Gordonia.*

Advantageously, said protein may be not obtainable by co-precipitation with any protein selected from the group consisting of the proteins of the *Corynebacterium, Nocardia, Rhodococcus* and *Gordonia* cells. More particularly, said protein may be not obtainable by
- lysing mycobacterial cells under non-denaturing conditions,
- contacting said lysed cells, or a protein extract thereof, with an affinity ligand or with an antibody (more particularly with an affinity ligand), which (is covalently linked or) binds to at least one bait protein binds to at least one bait protein and which co-precipitates proteins that are bound to said at least one bait protein, wherein said at least one bait protein is any protein selected from the group consisting of the proteins of the *Corynebacterium, Nocardia, Rhodococcus* and *Gordonia* cells, and
- isolating or purifying the protein from the proteins, which co-precipitate with said at least one bait protein.

Advantageously, said protein may be an enzyme that catalyzes the formation of a double bond in a meromycolic chain, more particularly in a mycobacterial meromycolic chain.

Said double bond formation may allow the biosynthesis of a full-size meromycolic chain, more particularly of a mycobacterial meromycolic chain, to produce alpha-mycolic acid(s), and optionally the oxygenated mycolic acid(s) such as epoxy-, methoxy- or keto-mycolic acid(s).

Said protein may have retained the enzymatic activity of a dehydratase (more particularly an activity of dehydration, more particularly an activity of dehydration and an activity of isomerization) on the meromycolic chain of mycobacterial mycolic acids or a biosynthetic intermediate of the meromycolic chain.

Said mycolic acid may for example be alpha-mycolic acid or an oxygenated form thereof, such as epoxy-mycolic acid, methoxy-mycolic acid or keto-mycolic acid. Said *Mycobacterium* may e.g., be a Non-Tuberculous Mycobacterium (NTM), such as *Mycobacterium smegmatis.*

Said protein may e.g., be an enzyme that catalyzes the dehydration, and optionally the isomerization, of

CH₃-(CH₂)_{15/17}-CH=CH-(CH₂)_{17/19}-COO-AcpM (I)

to produce

CH₃-(CH₂)_{15/17}-CH=CH-(CH₂)_{14/16}-CH=CH-CH₂-COO-AcpM (II),

or

CH₃-(CH₂)_{15/17}-CH=CH-(CH₂)_{14/16}-CH=CH-(CH₂)_{17/19}-COO-AcpM (III)

wherein AcpM is mycobacterial Acyl Carrier Protein (*cf.* Figure 6).

It may for example be the case of the HadD protein *of M. smegmatis.*

Said protein may e.g., be an enzyme that catalyzes the dehydration, and optionally the isomerization, of beta-hydroxyacyl-ACP to produce cis-3-enoyl-ACP, wherein ACP is Acyl Carrier Protein (*cf.* Figure 11).

Said protein may be an enzyme that catalyzes the dehydration, and optionally the isomerization, of beta-hydroxyacyl-AcpM to produce cis-3-enoyl-AcpM, wherein AcpM is mycobacterial Acyl Carrier Protein.

It may for example be the case of the HadD protein *of M. smegmatis.*

Said protein may e.g., be a protein, the amino acid sequence of which is chosen

from among SEQ ID NO: 1 and 10-16, more particularly from among SEQ ID NO: 1 and 10-13, more particularly SEQ ID NO: 1, or

from among SEQ ID NO: 2-9, more particularly from among SEQ ID NO: 2-8, more particularly SEQ ID NO: 2.

Please see Tables 1 and 2 in the examples below as well as the appended ST25 sequence listing.

Said protein may have retained the amino acid motif of SEQ ID NO: 49, more particularly of SEQ ID NO: 50. Alternatively or complementarily, said protein may have retained the amino acid motif of SEQ ID NO: 37, more particularly of SEQ ID NO: 38.

The amino acid motif of SEQ ID NO: 49 is EFXXAXXDXXPAHXXXXXA, wherein X at positions 3, 4, 6, 7, 9, 10, 14, 15, 16, 17 and 18 is any amino acid.

The amino acid motif of SEQ ID NO: 50 is EFSAAVKDDHPAHFDEAAA (and may be found e.g., in the HadD sequence *of M. smegmatis*)*.*

The amino acid motif of SEQ ID NO: 37 is FXXAXXDXXPAHXXXXXA, wherein X at positions 2, 3, 5, 6, 8, 9, 13, 14, 15, 16 and 17 is any amino acid.

The amino acid motif of SEQ ID NO: 38 is FSAAVKDDHPAHFDEAAA (and may be found e.g., in the HadD sequence *of M. smegmatis*)*.*

The amino acid motifs of SEQ ID NO: 49, 50, 37 and 38 may be viewed as particular HadD hydratase motifs.

Said protein may be obtainable (by isolation or purification) from *Mycobacterium smegmatis, M. avium, M. marinum, M. ulcerans, M. kansasii,* from a mycobacterium of the tuberculosis complex (*M*. *tuberculosis, M. bovis, M. africanum, M. microti, M. canettii, M. caprae, M. orygis, M. mungi and M. pinnipedii*)*,* or from *M. leprae.*

Said protein may comprise an alpha helix and a five-stranded antiparallel beta-sheet, and may further comprise two alpha helices forming a loop (*cf.* e.g., Figure 7B).

Said loop may comprise a dehydratase motif, more particularly a dehydratase motif of SEQ ID NO: 39, more particularly a dehydratase motif chosen from among SEQ ID NO: 40-48, for example a dehydratase motif of SEQ ID NO: 49 (e.g., a dehydratase motif of SEQ ID NO: 50).

The Molecular Weight (MW) of said protein may be of 19-20 kDa, e.g., 19.6 kDa.

The protein may be in crystalline or non-crystalline form.

The protein may be viewed as an enzyme, which, when it is not expressed in a *Mycobacterium,* induces at least one of the following effects:
- the effect of inhibiting, or slowing down, the growth of said *Mycobacterium,* more particularly when said Mycobacterium is placed in a liquid culture medium;
- the effect of altering the integrity of the envelope of said *Mycobacterium;*
- the effect of inhibiting the Fatty Acid Synthase type II (FAS-II) of said *Mycobacterium;*
- the effect of inhibiting the production of alpha-mycolic acids by said *Mycobacterium;*
- the effect of inhibiting the production by said *Mycobacterium* of oxygenated mycolic acid(s), such as epoxy-, methoxy- or keto-mycolic acid(s);
- the effect of inhibiting the capacity or propensity of the cells of said *Mycobacterium* to aggregate or sediment, more particularly when said cells are placed in a liquid culture medium;
- the effect of inducing a change of the morphology of the colony formed by the cells of said *Mycobacterium,* e.g., a change from a rough to a smooth phenotype (e.g., as shown on Figure 1), more particularly when said cells are placed on a solid culture medium;
- the effect of inhibiting the capacity or propensity of the cells of said *Mycobacterium* to assemble into a biofilm;
- the effect of altering the capacity of sliding motility of the cells of said *Mycobacterium;*
- the effect of increasing the sensitivity of said *Mycobacterium* to a temperature stress, e.g., to a temperature lower than 35°C, e.g., a temperature of 30°C;
- the effect of increasing the sensitivity of said *Mycobacterium* to a denaturing detergent, such as SDS;
- the effect of increasing the sensitivity of said *Mycobacterium* to the human immune system;
- the effect of decreasing the fitness of said *Mycobacterium;*
- the effect of decreasing the resistance of said *Mycobacterium* to an antimycobacterial compound or drug, more particularly to an anti-tuberculosis compound or drug, more particularly to at least one anti-tuberculosis compound or drug chosen from among rifampicin, pyrazinamide, isoxyl, thiacetazone, ethionamide and isoniazid; and
- the effect of increasing the sensitivity of said *Mycobacterium* to an antimycobacterial compound or drug, more particularly to an anti-tuberculosis compound or drug, more particularly to at least one anti-tuberculosis compound or drug chosen from among rifampicin, pyrazinamide, isoxyl, thiacetazone, ethionamide and isoniazid,
said *Mycobacterium* being more particularly
- a Non-Tuberculous Mycobacterium (NTM), such as *Mycobacterium smegmatis, M. avium, M. marinum, M. ulcerans, M. kansasii, M. abscessus, M. chelonae, M. fortuitum, M. xenopi, M. simiae* and *M. szulgai*; or
- a non-NTM mycobacterium, such as a mycobacterium of the tuberculosis complex (*M. tuberculosis, M. bovis, M. africanum, M. microti* and *M. canettii*)*,* or such as *M. leprae.*

The application also relates to a HadD ligand. More particularly, the application relates to a ligand, which specifically binds to the (HadD) protein of the application.

Said ligand may e.g., be a compound, which can be delivered intracellularly, more particularly into a *Mycobacterium* cell.

Said ligand may e.g., be a compound, which can bind to the surface or to components of the cell envelope, more particularly of the *Mycobacterium* cell.

Alternatively or complementarily, said ligand may e.g., be:
- an antibody, or
- a monoclonal antibody, or
- a fragment of (monoclonal) antibody, which has retained the antigen binding specificity of said (monoclonal) antibody, such as a Fab, Fab' or F(ab)2 fragment, or
- a fusion protein, which has retained the antigen binding specificity of said (monoclonal) antibody, such as a scFv, or
- a single-domain antibody (sdAb), or
- the variable domain of a sdAb,
wherein said product specifically binds the (HadD) protein of the application.

Said ligand (more particularly said (monoclonal) antibody) may be linked to a solid support, for example a bead, more particularly an affinity bead. Said ligand may be (covalently) linked to a tag, e.g. to an affinity purification tag.

The phrase "antibody" or "monoclonal antibody" includes conventional antibody (which comprises a heavy chain and a light chains) as well as single-domain antibody (sdAb; which, by contrast to a conventional antibody, is devoid of light chains and consists of a single monomeric variable antibody domain), such as a Heavy Chain Antibody (hcAb).

The phrase "antibody" or "monoclonal antibody" includes mono-, bi- or tri-specific antibodies.

The expression "fragment of (monoclonal) antibody, which has retained the antigen binding specificity" includes Fab, Fab' and F(ab)2 fragments (of a conventional Ab or mAb), as well as the variable domain of a sdAb or hcAb (VHH or nanobody).

Said antibody, antibody fragment or fusion protein may optionally be linked or bound to at least one detection label or marker or tag.

The application also relates to a hybridoma, which produces a monoclonal antibody of the application.

The application also relates to a polypeptide, the amino acid sequence of which is the sequence of a fragment of the (HadD) protein of the application, wherein said polypeptide is specifically bound by the ligand of the application, more particularly by the (monoclonal) antibody of the application.

The application also relates to a protein complex, which comprises the (HadD) protein of application or the polypeptide of the application.

The application also relates to a protein crystal, which diffracts X-rays, wherein the protein of said crystal comprises the (HadD) protein of the application or the polypeptide of the application.

The structure of the protein in said crystal may e.g., be as shown on Figure 7B.

The application also relates to a method to identify antimycobacterial compound(s), wherein said method comprises
- testing candidate compound(s) for binding to the (HadD) protein of the application or to the polypeptide of the application or to the protein complex of the application or to the protein crystal of the application, and
- selecting the compound(s) that binds(bind) to said protein, polypeptide, protein complex or protein crystal, more particularly compound(s) that specifically binds(bind) to said protein, polypeptide, protein complex or protein crystal, wherein selecting said compound(s) identifies it(them) as antimycobacterial compound(s).

The application also relates to a method to identify antimycobacterial compound(s), wherein said method comprises
- testing candidate compound(s) for competition to binding to the (HadD) protein of the application or to the polypeptide of the application or to the protein complex of the application or to the protein crystal of the application, more particularly candidate compound(s) which competes(compete) with a (HadD) ligand, such as an anti-HadD monoclonal antibody, for said binding, and
- selecting the compound(s) that competes(compete) for binding to said protein, polypeptide, protein complex or protein crystal, more particularly compound(s) that specifically binds(bind) to said protein, polypeptide, protein complex or protein crystal, wherein selecting said compound(s) identifies it(them) as antimycobacterial compound(s). The application also relates to a method to identify antimycobacterial compound(s), wherein said method comprises
- testing candidate compound(s) for binding to the (HadD) protein of the application and for inhibiting an enzymatic activity of said protein (more particularly a dehydratase and/or isomerase activity of said protein), and
- selecting the compound(s), which binds(bind) to said protein, more particularly compound(s) that specifically binds(bind) to said protein, and which inhibits(inhibit) said activity, wherein selecting said compound(s) identifies it(them) as antimycobacterial compound(s).

Said method may e.g., be an *in vitro* and/or *in silico* method, more particularly an *in vitro* method.

Said selected compound(s) may e.g., be compound(s), which inhibits(inhibit) a dehydratase activity of said protein or polypeptide, or an isomerase activity of said protein or polypeptide, or a dehydratase and isomerase activity of said protein or polypeptide.

Said selected compound(s) may e.g., be compound(s), which when placed into contact with a *Mycobacterium,* induces(induce) at least one of the following effects:
- the effect of inhibiting, or slowing down, the growth of said *Mycobacterium,* more particularly when said *Mycobacterium* is placed in a liquid culture medium;
- the effect of altering the integrity of the envelop of said *Mycobacterium;*
- the effect of inhibiting the Fatty Acid Synthase type II (FAS-II) of said *Mycobacterium;*
- the effect of inhibiting the production of alpha-mycolic acids by said *Mycobacterium;*
- the effect of inhibiting the production by said *Mycobacterium* of oxygenated mycolic acid(s), such as epoxy-, methoxy- or keto-mycolic acid(s);
- the effect of inhibiting the capacity or propensity of the cells of said *Mycobacterium* to aggregate or sediment, more particularly when said cells are placed in a liquid culture medium;
- the effect of inducing a change of the morphology of the colony formed by the cells of said *Mycobacterium,* e.g., a change from a rough to a smooth phenotype (e.g., as shown on Figure 1), more particularly when said cells are placed on a solid culture medium;
- the effect of inhibiting the capacity or propensity of the cells of said *Mycobacterium* to assemble into a biofilm;
- the effect of altering the capacity of sliding motility of the cells of said *Mycobacterium;*
- the effect of increasing the sensitivity of said *Mycobacterium* to a temperature stress, e.g., to a temperature lower than 35°C, e.g., a temperature of 30°C;
- the effect of increasing the sensitivity of said *Mycobacterium* to a denaturing detergent, such as SDS;
- the effect of increasing the sensitivity of said *Mycobacterium* to the human immune system;
- the effect of decreasing the fitness of said *Mycobacterium;*
- the effect of decreasing the resistance of said *Mycobacterium* to an antimycobacterial compound or drug, more particularly to an anti-tuberculosis compound or drug, more particularly to at least one anti-tuberculosis compound or drug chosen from among rifampicin, pyrazinamide, isoxyl, thiacetazone, ethionamide and isoniazid; and
- the effect of increasing the sensitivity of said *Mycobacterium* to an antimycobacterial compound or drug, more particularly to an anti-tuberculosis compound or drug, more particularly to at least one anti-tuberculosis compound or drug chosen from among rifampicin, pyrazinamide, isoxyl, thiacetazone, ethionamide and isoniazid.

The application also relates to a composition, pharmaceutical composition, or drug, which comprises at least one compound, which (specifically) binds to the (HadD) protein of the application, more particularly at least one ligand of the application.

The composition, pharmaceutical composition, or drug of the application may further comprise at least one other compound or drug, i.e., at least one compound or drug, which is other than (i.e., structurally different from) said compound that (specifically) binds to the (HadD) protein of the application, more particularly other than (i.e., structurally different from) said at least one ligand of the application. Said at least one other compound or drug may advantageously be chosen from among the anti-mycobacterial compounds or drugs and the anti-tuberculosis compounds or drugs. Said at least one anti-tuberculosis compound or drug may e.g., be chosen from among rifampicin, pyrazinamide, isoxyl, thiacetazone, ethionamide and isoniazid.

The application also relates to a compound, which (specifically) binds to the (HadD) protein of the application, more particularly to the ligand of the application or to the composition, for use in the treatment of mycobacterial diseases.

The application also relates to the pharmaceutical composition, or drug of the application, for use in the treatment of mycobacterial diseases.

Said mycobacterial diseases may e.g., be a Non-Tuberculous Mycobacterium (NTM) infection, a nosocomial infection, the bacterial infection of an immunocompromise patient, a non-NTM infection, tuberculosis or leprosy, more particularly a NTM infection, tuberculosis or leprosy, more particularly a NTM infection or leprosy.

NTM may more particularly be one or several from among *Mycobacterium smegmatis, M. avium, M. marinum, M. ulcerans, M. kansasii, M. abscessus, M. chelonae, M. fortuitum, M. xenopi, M. simiae* and *M. szulgai.*

Non-NTM may more particularly be one or several from among the mycobacteria of the tuberculosis complex (*M*. *tuberculosis, M. bovis, M. africanum, M. microti, M. canettii, M. caprae, M. orygis, M. mungi and M. pinnipedii*)*,* and *M. leprae,* more particularly one or several from among *M. tuberculosis, M. bovis, M. africanum, M. microti* and *M. canettii,* more particularly at least *M. tuberculosis.*

The application also relates to a nucleic acid, the nucleotide sequence of which consists of a sequence, which codes for the (HadD) protein of the application or for the polypeptide of the application.

More particularly, the application relates to a nucleic acid, which codes for a protein, the amino sequence of which is chosen from among
SEQ ID NO: 1 and 10-16, more particularly SEQ ID NO: 1 and 10-13; or
SEQ ID NO: 2-9, more particularly SEQ ID NO: 2-8.

More particularly, the application relates to a nucleic acid, the coding sequence of which is a nucleotide sequence chosen from among
SEQ ID NO: 17 and 26-32, more particularly from among SEQ ID NO: 17 and 26-29, or from among
SEQ ID NO: 18-25, more particularly from among SEQ ID NO: 18-24.

The application also relates to a recombinant nucleic acid vector, which carries a nucleic acid insert, more particularly for expression of said nucleic acid insert, wherein said nucleic acid insert comprises the nucleic acid of the application.

The application also relates to a nucleic acid vector, which recombinantly codes for the expression of a protein, wherein said protein is the protein of any one of claims 1-6.

The application also relates to a genetically engineered cell, which recombinantly comprises (more particularly which recombinantly expresses) the nucleic acid of the application or the recombinant nucleic acid vector of the application. Said genetically engineered cell may e.g., be other than a *Mycobacterium* cell: it can e.g., be an *E. coli* cell (*cf.* example 2 below).

The application also relates to a genetically modified *Mycobacterium* cell, more particularly an attenuated *Mycobacterium* cell, which has been engineered to inhibit or knock out the expression of a protein, wherein said protein is the (HadD) protein of the application.

Said cell may e.g., be
- a cell of a Non-Tuberculous Mycobacterium (NTM), such as *Mycobacterium smegmatis, M. avium, M. marinum, M. ulcerans, M. kansasii, M. abscessus, M. chelonae, M. fortuitum, M. xenopi, M. simiae* and M. *szulgai*; or
- a cell of a non-NTM mycobacterium, such as a mycobacterium of the tuberculosis complex (*M*. *tuberculosis, M. bovis, M. africanum, M. microti, M. canettii, M. caprae, M. orygis, M. mungi and M. pinnipedii*)*,* or such as *M. leprae.*

The genetically modified *Mycobacterium* cell of the application may have been engineered by full or at least partial gene deletion, and may not comprise any nucleic acid coding for the (HadD) protein of the application.

For example, the genetically modified *Mycobacterium* cell comprises the nucleic acid of SEQ ID NO: 34 instead of the nucleic acid of SEQ ID NO: 17 (*M. smegmatis hadD* knock-out; *cf.* example 1 below).

For example, the genetically modified *Mycobacterium* cell comprises the nucleic acid of SEQ ID NO: 33 instead of the nucleic acid of SEQ ID NO: 18 (*M. tuberculosis hadD* knock-out; *cf.* example 4 below).

The application also relates to the genetically modified *Mycobacterium* cell of the application, for use in the treatment of mycobacterial diseases.

Said mycobacterial disease may e.g., be a Non-Tuberculous Mycobacterium (NTM) infection, a nosocomial infection, the (myco)bacterial infection of an immunocompromise patient, a non-NTM infection, tuberculosis or leprosy, more particularly a NTM infection, tuberculosis or leprosy, more particularly a NTM infection or tuberculosis, more particularly a NTM infection.

NTM may more particularly be one or several from among *Mycobacterium smegmatis, M. avium, M. marinum, M. ulcerans, M. kansasii, M. abscessus, M. chelonae, M. fortuitum, M. xenopi, M. simiae* and *M. szulgai.*

Non-NTM may more particularly be one or several from among the mycobacteria of the tuberculosis complex (*M*. *tuberculosis, M. bovis, M. africanum, M. microti, M. canettii, M. caprae, M. orygis, M. mungi and M. pinnipedii*)*,* and *M. leprae,* more particularly one or several from among *M. tuberculosis, M. bovis, M. africanum, M. microti* and *M. canettii,* more particularly at least *M. tuberculosis.*

In the application, unless specified otherwise or unless a context dictates otherwise, all the terms have their ordinary meaning in the relevant field(s).

The term "comprising", which is synonymous with "including" or "containing", is openended, and does not exclude additional, unrecited element(s), ingredient(s) or method step(s), whereas the term "consisting of" is a closed term, which excludes any additional element, step, or ingredient which is not explicitly recited.

The term "essentially consisting of" is a partially open term, which does not exclude additional, unrecited element(s), step(s), or ingredient(s), as long as these additional element(s), step(s) or ingredient(s) do not materially affect the basic and novel properties of the invention.

The term "comprising" (or "comprise(s)") hence includes the term "consisting of" ("consist(s) of"), as well as the term "essentially consisting of" ("essentially consist(s) of"). Accordingly, the term "comprising" (or "comprise(s)") is, in the application, meant as more particularly encompassing the term "consisting of" ("consist(s) of"), and the term "essentially consisting of" ("essentially consist(s) of").

In an attempt to help the reader of the application, the description has been separated in various paragraphs or sections. These separations should not be considered as disconnecting the substance of a paragraph or section from the substance of another paragraph or section. To the contrary, the description encompasses all the combinations of the various sections, paragraphs and sentences that can be contemplated.

### EXAMPLES

**EXAMPLE 1:** HadD, a novel dehydratase-like protein of the fatty acid synthase type II of the NonTuberculous Mycobacteria (NTM) is required for the biosynthesis of the alpha- and epoxy-mycolic acids and crucial for bacterial envelope properties, fitness, population organization and motility.

We identified a new enzyme of the FAS-II system, *i.e.,* MSMEG_0948 (HadD). HadD was identified as an abundant homologue of FAS-II dehydratase subunits, HadA and HadC and physically interacting with the HadAB enzyme. The impact of the depletion in HadD on both the physiology and the MA biosynthesis was examined. The data convincingly leaded to the conclusion that HadD function consists of forming a double bond at the proximal position of the meromycolic chain of MAs, which is absolutely required for the production of both α- and epoxy-MAs *of M. smegmatis.*

### EXPERIMENTAL PROCEDURES

### Pull-downs

For pull-down experiments using HadAB enzyme as a bait, *a M. smegmatis* strain deleted for the endogenous *hadABC* operon (*MSMEG*_*1340*-*1341*-*1342*) (Jamet, Slama et al. 2015) was complemented by the plasmid pKW08::*gfp-hadABC.* The latter was constructed by cloning the *hadABC* operon of *M. tuberculosis* H37Rv (*Rv0635-0636-0637*) in the tetracycline-inducible vector pKW08::*gfp* (Williams, Joyce et al. 2010; Plocinski, Laubitz et al. 2014) between BamH1 and HindIII sites, downstream of the enhanced Green Fluorescent Protein (eGFP) tag followed by the Tobacco Etch Virus (TEV) cleavage site. The control strain used for these experiments was *M. smegmatis* Δ*hadABC*/pKW08::*gfp* complemented by the pGBT::*hadABC* vector (Jamet, Slama et al. 2015). The resulting strains were grown in MIDDLEBROOK 7H9 broth (DIFCO) supplemented with 50 µg/ml hygromycin, 10 % (w,v) ADC and 0.2 % (v/v) glycerol, and the production of the protein(s) was induced by adding 50 ng/ml tetracycline (SIGMA) during the exponential phase. The affinity purification method used was adapted from a published protocol (Plocinski, Laubitz et al. 2014). Bacteria were centrifuged at 10,000 g for 30 min at 4 °C, resuspended in lysis buffer: 50 mM potassium phosphate buffer pH 7.6, 100 mM NaCl, 1 mM DTT, 2 mM AEBSF (EUROMEDEX), 1 % (v,v) TRITON X-100, 5 µg/ml DNAse I and 5 µg/ml RNase A, and lysed using a BIORUPTOR PICO (DIAGENODE) for 20 cycles (30 sec on, 30 sec off). The lysate was clarified by centrifugation at 20,000 g for 20 min at 4° C and incubated in the presence of GFP-Trap®_MA magnetic beads (CHROMOTEK) for 3 h at 4°C. After four washes with 10 mM Tris pH 7.6, 150 mM NaCl, 0.1 % (v,v) TRITON X-100, proteins were eluted by cleavage using the AcTEV™ protease (FISHER) following the manufacturer protocol.

### Sample preparation for proteomics analysis.

Cysteine residues of eluted proteins were reduced by addition of 13 mM final of β-mercaptoethanol in 50 mM Tris-HCl pH6.8, 5% glycerol, 1% SDS, 0.05% bromophenol blue for 10 min at 95°C (30 min for cross-linked proteins), and alkylated by addition of chloroacetamide at a final concentration of 45 mM for 30 min at room temperature in the dark. Each protein sample was loaded onto 12% SDS-PAGE gel. The electrophoretic migration was stopped as soon as the protein sample entered the separating gel. The gel was stained with Instant Blue (EUROMEDEX), and a single band containing the whole sample, was cut. Gel slice was washed for 15 min at 37°C first with 100 mM ammonium bicarbonate then several cycles with 100 mM ammonium bicarbonate:acetonitrile (1:1) until the gel pieces were colorless, and finally in 100% acetonitrile. The proteins were then digested overnight at 37°C with 0.6 µg of modified sequencing grade trypsin (PROMEGA) in 50 mM ammonium bicarbonate. The resulting peptides were extracted from the gel by incubation in 50 mM ammonium bicarbonate for 15 min at 37°C followed by two incubations in 10% formic acid:acetonitrile (1:1) for 15 min at 37°C. The resulting peptides were dried in a SPEEDVAC, and resuspended in 2% acetonitrile, 0.05% TFA before being subjected to nanoLC-MS/MS analysis.

### NanoLC-MS/MS Analysis.

Peptides were analyzed by nanoLC-MS/MS using an ULTIMATE 3000 RSLCnano system (DIONEX, Amsterdam, The Netherlands) coupled to a Q-EXACTIVEPLUS mass spectrometer (THERMOSCIENTIFIC, Bremen, Germany). 2 µl of each sample were loaded onto a C-18 precolumn (300-µm inner diameter x 5 mm, DIONEX) at 20 µl/min in 5% acetonitrile, 0.05% Trifluoroacetic Acid (TFA). After 5 min of desalting, the precolumn was switched online with the analytical C-18 column (75 µm inner diameter x 50 cm; in-house packed with REPROSIL C18) equilibrated in 95% solvent A (5% acetonitrile, 0.2% formic acid) and 5% solvent B (80% acetonitrile, 0.2% formic acid). The peptides were eluted using a 5 to 50% gradient of solvent B for 60 min at 300 nl/min flow rate. The mass spectrometer was operated in data-dependent acquisition mode with the XCALIBUR software. Survey MS scans were acquired in the ORBITRAP on the 350-1500 *m*/*z* range with a resolution of 70000. The 10 most intense ions per survey scan were selected for HCD fragmentation and resulting fragments were analyzed at a resolution of 17500 in the ORBITRAP.

### Protein identification, quantification and statistical analysis.

Raw MS files were analyzed by MAXQUANT version 1.5.5.1. Data were searched with the ANDROMEDA search engine against *Mycobacterium smegmatis* entries of the SWISSPROT-TREMBL protein database (UNIPROTKB/SWISS-PROT Knowledgebase release 2017_03, *M. smegmatis* MC2 taxonomy, 8805 entries) and a list of potential contaminant sequences provided in MAXQUANT1.5.5.1. The search included methionine oxidation and protein N-terminal acetylation as variable modifications, and carbamidomethylation of cysteine as a fixed modification. Validation was performed through a false discovery rate set to 1% at protein and PSM level determined by target-decoy search in MAXQUANT (with a minimum length of 7 amino acids). Specificity of trypsin digestion was set for cleavage after lysine or arginine, and two missed cleavages were allowed. The precursor mass tolerance was set to 20 ppm for the first search and 4.5 ppm for the main ANDROMEDA database search. The mass tolerance in MS/MS mode was set to 20 ppm. The minimal ratio count was set to 1 for calculation of LFQ intensities. The data set contains mass spectrometry results from the analysis of 4 biological replicates. The statistical proteomics analysis was performed using the PERSEUS 1.5.3.0 software on the LFQ intensities from the "proteinGroups" table of MAXQUANT. Two-sided Student t-test, variance correction and permutation-based false discovery rate (FDR) control were performed in Perseus in order to find significantly enriched proteins (FDR < 5%).

### Sequence analyses and homology modeling.

Analyses of genome sequences were done via the SMEGMALLIST (Kapopoulou, Lew et al. 2011) and the NCBI web sites (www.ncbi.nlm.nih.gov/genomes/lproks.cgi). Sequence alignments were performed using BLAST and CLUSTAL OMEGA software (Altschul, Madden et al. 1997; Sievers, Wilm et al. 2011), with default parameters. Structure prediction, homology modeling and model evaluation were performed through the @TOME 2.3 Platform (Pons and Labesse 2009). Briefly, the structural alignment research against PDB was done using HHSEARCH. The 3D model was built using the crystal structure of *M. tuberculosis* HadA protein (4RLJ) (41% sequence identity with MSMEG_0948) as a template in the program MODELLER V9 (Sali and Blundell 1993). The model was evaluated through the internal MODELLER energy score and MODELLER, QMEAN, VERIFY3D, DOPE, DFIRE AND ERRAT software. The good scores obtained (e.g. MODELLER: 604.39; QMEAN: 0.51) indicated the good quality of the model.

### Construction of the deletion mutant and the complemented strain, and culture conditions.

The *M. smegmatis ΔMSMEG_0948* mutant was constructed from *M. smegmatis* mc² 155 strain (strain ATCC 700084; genome sequence CP009494) by the recombineering system (van Kessel and Hatfull 2007) with some method modifications as described previously (Jamet, Quentin et al. 2015). This resulted in the replacement of the target gene by a streptomycin resistance cassette (SEQ ID NO: 34), as checked by PCR and sequencing. For complementation, a wt copy of *MSMEG*_*0948* gene was introduced between the *Not*I and *Eco*RI restriction sites of the integrative plasmid pUC-Gm-Int (Parish 2000) and the resulting plasmid was used to transform *M. smegmatis ΔMSMEG_0948* strain.

Both the parent *M. smegmatis* mc² 155 and *M. smegmatis* Δ*MSMEG_0948* strains were also transformed by the empty pUC-Gm-Int plasmid.

The standard culture conditions were at 37°C either under shaking (190 rpm) in Middlebrook 7H9 broth (DIFCO) supplemented with 0.2% glycerol and gentamycin (5 µg/ml), or on plates of MIDDLEBROOK 7H10 medium (DIFCO) supplemented with 0.2% glycerol and gentamycin (10 µg/ml).

### Phenotyping assays.

Growth curves were realized by measuring the OD at 600 nm of liquid cultures incubated at 37°C under shaking (190 rpm) in MIDDLEBROOK 7H9 broth (DIFCO) supplemented by 0.2% glycerol, 0.05% (w/v) Tween-80, 10% ADC (DIFCO) and 5 µg/ml gentamycin. For biofilm formation (Kulka, Hatfull et al. 2012), exponential precultures (OD₆₀₀ ∼ 1) in the above medium were diluted (1:100) in standard Sauton's medium containing gentamycin (5 µg/ml). Then 5 ml aliquots were dispensed into 6-well plates and incubated at 37°C for 6 or 10 days. For sedimentation assays, liquid cultures incubated at 37°C under shaking (190 rpm) in MIDDLEBROOK 7H9 broth supplemented with 0.2% glycerol and gentamycin (5 µg/ml) were stopped at an OD₆₀₀ of 4-5 and then kept unshaken at room temperature for 15 min. For Congo red, sliding motility, colony morphology and SDS, drug and temperature susceptibility assays, bacteria were grown in MIDDLEBROOK 7H9 broth containing 0.2% glycerol, 0.05% (w/v) TWEEN-80 and gentamycin (5µg/ml) until an OD₆₀₀ of 4-5 and the different precultures were then adjusted to the same OD. For colony morphology and Congo red assays, 5 µl preculture aliquots were spotted on MIDDLEBROOK 7H10 medium (DIFCO) containing 0.2% glycerol supplemented with 100 µg/ml Congo red (SIGMA) when required, and incubated at 37°C for 3 days. For the following experiments, the precultures were serially diluted. For sliding motility assays, 10 µl aliquots of each dilution were spotted onto semi-solid MIDDLEBROOK 7H9 broth containing 0.3% agar without additional carbon source and incubated at 37°C for a week. For temperature/drug susceptibility assays, 5 µl aliquots of each dilution were spotted onto MIDDLEBROOK 7H10 medium. When required, the tested drug or detergent was added to the medium: rifampicin (2 µg/ml), isoniazid (5 µg/ml), isoxyl (10 µg/ml), thioacetazone (5 µg/ml), ethionamide (5 µg/ml), pyrazinamide (10 µg/ml), ethambutol (1 µg/ml) or SDS (0.005 or 0.01%). Cultures were incubated for 3 days at 37°C (for drug/detergent testing) as well as 30°C and 42°C (for temperature testing).

### Lipid extractions and (HP)TLC analyses

The total extractable lipids were extracted from wet bacterial pellets by three successive extractions using distinct mixtures of CHCl₃:CH₃OH (1:2, 1:1 and 2:1, v/v). The fractions were pooled, washed with water, and dried. For TLC analysis, equivalent weights of total lipid fraction from each strain were spotted on silica gel 60 plates (Merck), which were developed either in CHCl₃:CH₃OH (9:1, v/v) or in CHCl₃:CH₃OH:H₂O (65:25:4, v/v/v), as indicated. Compounds were revealed by spraying with 0.2% (w/v) anthrone in concentrated H₂SO₄ followed by heating at 100°C.

MAs were extracted both from the delipidated bacterial residues and the total extractable lipids after saponification as previously described (Laneelle, Launay et al. 2012), dried and weighted. The dry weights of MAs were normalized by the dry weights of the bacterial residues. MAs were methylated with diazomethane. The relative abundance of the different MAME classes from each strain was determined by loading a fixed amount (5 µg) of MA mixture onto a HPTLC silica gel 60 plate (Merck) with a CAMAG ATS4 apparatus. The plate was developed in petroleum ether/diethyl ether (9:1, v/v) using a CAMAG ADC2 device and stained by immersion in 10% (w/v) CuSO₄ (in H₃PO₄:CH₃OH:H₂O, 8:5:87, v/v/v) with a CAMAG CID3 apparatus, followed by heating at 150°C for 20 min. The MAMEs were quantified by absorption measurement at 400 nm with a CAMAG Scanner 3 device using WINCATS software. For TLC analyses of MAMEs, equivalent weights of compounds were spotted on silica gel 60 plates (Merck), which were developed in dichloromethane and revealed by spraying by CuSO₄ solution (see above) followed by heating at 100°C.

### Purification of TMM/TDM, and product X

TMM, TDM and compound X were separated from other lipids of the total lipid fractions by anion-exchange chromatography with a quaternary methylammonium column (PALL) using increasing percentages (0 to 100%) of CH₃OH in CHCl₃, and analyzed by TLC using silica gel 60 plates (MERCK) developed in CHCl₃:CH₃OH:H₂O (65:25:4, v/v/v). The purification was achieved by preparative TLC using silica gel 60 plates (MERCK) developed in the same solvent. The compounds of interest were then scraped off and extracted from silica gel three times with CHCl₃/CH₃OH (7:3 v/v).

### MALDI-TOF MS and NMR spectroscopy

Matrix assisted laser desorption ionization-time of flight mass spectrometry (MALDI-TOF MS) and MS/MS analyses were performed in the positive ionization and reflectron mode, using the 5800 MALDI-TOF/TOF Analyzer (APPLIED BIOSYSTEMS/ABSCIEX) equipped with a Nd:YAG laser (349 nm wavelength). MS and MS/MS spectra were acquired in positive ionization mode with a total of 2,500 shots at a fixed laser intensity of 4,000 (instrument-specific units) and 400-Hz pulse rate for MS, and a total of 5000 shots at a fixed laser intensity of 6000 (instrument-specific units) and 1000-Hz pulse rate for MS/MS. For MS/MS data acquisition, the fragmentation of selected precursors ions was performed at a collision energy of 1kV using air as collision gaz. Lipid samples were dissolved in chloroform and were directly spotted onto the target plate as 0.5 µl droplets, followed by the addition of 0.5 µl of matrix solution [10 mg/ml of 2,5-dihydroxybenzoic acid (SIGMA-ALDRICH) in CHCl₃/CH₃OH (1:1, v/v)]. Samples were allowed to crystallize at room temperature. MS data were acquired using the instrument default calibration.

1D and 2D ¹H-NMR, ¹H-¹H-COSY, ¹H-¹H-TOCSY (100 ms), ¹H-¹³C-HSQC and ¹H-¹³C-HMBC experiments were recorded at 298° K using a 600-MHz BRUKER AVANCE III spectrometer (BRUKER BIOSPIN) equipped with a TCI cryoprobe. Purified compound X was dissolved in CDCl₃/CD₃OD (8:2, v/v). Chemical shifts were referenced to CDCl₃ (δH 7.26 ppm and δC 77 ppm).

### RESULTS

### MSMEG_0948 protein interacts with HadAB enzyme of the FAS-II system

The (3*R*)-hydroxyacyl-ACP dehydratases HadAB and HadBC are two main components of the mycobacterial FAS-II system, HadA and HadC being the substrate-binding subunits and HadB the catalytic subunit (Sacco, Covarrubias et al. 2007; Biswas, Dutta et al. 2015). Accordingly, HadB is essential for the MA biosynthesis and the survival of *M. smegmatis* and *M. tuberculosis* (Sacco, Covarrubias et al. 2007; Jamet, Slama et al. 2015). The viability of a *M. smegmatis hadA hadC* double mutant maintaining the production of all MA types suggested the possible existence of a protein with a redundant function (Jamet, Slama et al. 2015). With the aim of discovering novel partner proteins of the HadAB dehydratase of the FAS-II system, pull-down experiments were performed from *M. smegmatis* bacterial lysates using HadA as a bait. For that, *hadABC* operon was expressed, with a enhanced Green Fluorescent Protein (eGFP) tag at the N-terminus of HadA, in a *M. smegmatis* strain deleted for the endogenous *hadABC* genes. The bait and interacting proteins were trapped using anti-GFP magnetic beads and, after extensive washes, released in mild conditions. The elution fractions, analyzed by nanoLC-MS/MS after trypsin digestion, displayed a strong and significant enrichment in HadB (Fig. 12), the second subunit of the HadAB enzyme. Strikingly, an equivalent enrichment was observed for an unknown protein, MSMEG_0948. This shows the occurrence of a highly specific physical interaction between HadAB enzyme and MSMEG_0948 protein.

MSMEG_0948 has a theoretical mass of 19.6 kDa and is annotated as "conserved hypothetical protein" (http://mycobrowser.epfl.ch/smegmalist.html; (Kapopoulou, Lew et al. 2011).

MSMEG_0948 represents the closest mycobacterial homologue of HadA (41% identity) and HadC (35% identity) subunits of the FAS-II dehydratases HadAB and HadBC, while it is much more distant from HadB (Fig. 7A).

Structural homology modelling predicts that a MSMEG_0948 monomer would organize, like HadA and HadC, as a 'single hot dog' fold made of a central *α*-helix (*α3*) wrapped into a five-stranded antiparallel *β*-sheet (Fig. 7B). Above this fold would sit a solvent-exposed loop corresponding to the "overhanging segment" first described in the (*R*)-hydratase from *Aeromonas caviae* (Hisano, Tsuge et al. 2003). This loop bears a degenerate hydratase 2 motif `F-x-x-a-x-x-D-x-x-P-x-H-x-x-x-x-x-A' (SEQ ID NO: 37) containing both catalytic Asp (D34) and His (H39) residues (Fig. 7B). This strongly suggests that MSMEG_0948 protein would be a (*R*)-specific hydratase/dehydratase related to the hydratase 2 subfamily, like HadAB and HadBC enzymes. We named it "HadD". We found that it is ubiquitous among all the sequenced mycobacterial genomes but absent from the other mycolic acid-producing genera of the *Corynebacteriales* order, such as *Corynebacterium, Nocardia, Rhodococcus,* and *Gordonia.* This represents an important discrepancy with HadAB enzyme, which is well conserved in these genera, and is reminiscent from HadC subunit behavior (Sacco, Covarrubias et al. 2007).

### MSMEG_0948 inactivation affects the bacterial cell surface and envelope properties

A *M. smegmatis MSMEG*_*0948* deletion mutant was constructed to determine the importance of HadD in the physiology of the bacterium. The viability of the strain showed that *MSMEG*_*0948* is a non-essential gene in *M. smegmatis* axenic culture. The ability of mycobacteria to multiply, assemble and colonize different kinds of surfaces in the environment as well as in the host plays a determinant role on their pathogenicity. The *M. smegmatis MSMEG_0948*-deficient bacteria displayed a reduced growth as compared to the wt strain in planktonic cultures (Fig. 1). Moreover, they sedimented more slowly in the absence of tween (Fig. 1), indicating that they aggregated to a lesser extent. On solid medium, inactivation of *MSMEG*_*0948* gene resulted in a drastic change of the colony morphology from a rough into a smooth phenotype. The mutant bacteria had also lost their capacity to assemble into biofilms, a successful strategy developed to survive under stress and hostile conditions. In contrast, *M. smegmatis* Δ*MSMEG_0948* strain exhibited a significantly increased sliding motility (Fig. 1), a flagellum-independent spreading mechanism of mycobacteria allowing their translocation on solid surfaces and probably crucial for colonization (Martinez, Torello et al. 1999). This phenomenon could be a consequence of a higher hydrophobicity of the mutant bacterial cell surface, which plays a major role in the sliding motility. Consistent with this, in contrast to the wt strain, colonies of the *MSMEG_0948*-deficient bacteria were intensely stained with Congo red, an hydrophobic dye that binds to lipoproteins or lipids present on the mycobacterial surface (Cangelosi, Palermo et al. 1999), resulting in a profound change of the colony morphology (Fig. 1).

The impact of *MSMEG_0948* gene inactivation on the response of mycobacteria to different stresses was then examined. The mutant strain displayed a marked sensitivity to low temperature (30°C) and to the detergent SDS as compared to the wt strain (Fig. 2A, 2B). It was also more sensitive than the wt strain to the first line TB drug pyrazinamide that targets the RNA translation process, as well as to the drugs isoniazid, ethionamide, isoxyl, thiacetazone and ethambutol, which inhibit the mycobacterial cell wall biogenesis (Fig. 2C). Moreover, it appeared hyper-susceptible to rifampicin, a large polyketide that blocks the DNA-dependent RNA synthesis. Thus, the modification of the level of sensitivity concerns a wide range of drugs with distinct sizes and degrees of hydrophobicity.

These data altogether reveal an upheaval of both the bacterial cell surface and the envelope properties of *M. smegmatis* upon *MSMEG*_*0948* inactivation, with consequences on the organization and motility of the bacterial populations and the envelope integrity and permeability. The complementation of the mutant strain by a wt *MSMEG*_*0948* copy, expectedly resulting in intermediate phenotypes (Fig. 1 and 2A-2C), showed that these phenomena are directly linked to *MSMEG*_*0948* deficiency.

### Mutation of MSMEG_0948 abolishes the production of cell-wall linked α- and epoxy-MAs

*M. smegmatis* produces three main types of mycolic acids (MAs): di-unsaturated compounds called α-MAs, shorter monounsaturated compounds called α'-MAs, and monounsaturated epoxydic compounds called epoxy-MAs (Fig. 3A). The impact of *MSMEG*_*0948* gene inactivation on the biosynthesis of mycolic acids (MAs) was examined. The total content of cell-wall linked MAs (that correspond to 93% of the MAs) remained constant after gene inactivation and represented 6.4 ±0.4 % of the delipidated bacteria dry weight. In contrast, the HPTLC MA profile changed dramatically, and the presence of α-and epoxy-mycolic acid methyl esters (MAMEs) could hardly be detected (Fig. 3B, 3C). This conclusion was supported by the MALDI-TOF MS analysis of the total MA mixture (Fig. 3D). *M. smegmatis* Δ*MSMEG_0948* only produces α'-MAs. This is the first description of such a mycobacterial strain. The partial recovery of the wt profile in the complemented strain indicates that this phenotype is directly linked to the lack of *MSMEG*_*0948* gene. The structures of the α'-MAs from the mutant strain are identical to those of the wt strain, as shown by MALDI-TOF MS and ¹H-NMR analyses (Fig. 3D, Fig. 8A-8B).

These data demonstrate that MSMEG_0948 protein plays an essential role in the biosynthesis of α- and epoxy-MAs, and is dispensable for α'-MA production.

### Disruption of the glycolipid content induced by MSMEG_0948 mutation

While the total amount of extractable lipids remained similar after inactivation of *MSMEG_0948,* significant qualitative changes were observed in their profile (Fig. 4A, 4B). The MA-containing lipids, i.e. the trehalose monomycolate (TMM) and the trehalose dimycolate (TDM), displayed smaller *R*_{f}. These compounds, called TMMΔ and TDMΔ, were analyzed by MALDI-TOF MS (Fig. 4C, 4D) and MALDI-TOF-TOF tandem MS (Fig. 9). The spectra corresponded to series of TMM and TDM homologs bearing only α'-mycoloyl chains. Consistent with this, the TLC profile showed the absence of α- and epoxy-MAs in the total extractable lipids (Fig. 5A). The MALDI-TOF mass spectra indicated that the TMMΔ and TDMΔ were also present in the wt strain (Fig. 4C, 4D), although in minority, as shown by TLC (Fig. 4A, 4B). Interestingly, *MSMEG*_*0948* deletion induced a doubling of the MA content coming from the extractable lipids (Fig. 5B) resulting in an increase of TDM quantity, possibly to compensate the loss of the longer chain MAs.

It is noteworthy that additional modifications of the extractable lipid profile were observed in the mutant strain. They included a marked decrease of the polar GPLs and the occurrence of a new compound (X; Fig. 4A, 4B). A COSY ¹H-¹H NMR analysis, confirmed by an HSQC ¹³C-¹H NMR analysis, revealed that the latter is a 2,3-diacyl trehalose. MALDI-TOF and MALDI-TOF/TOF MS analyses showed that compound X corresponds to a series of 2-fatty acyl,3-phleate trehalose homologs bearing a regular-size fatty acyl chain (C₁₄-C₁₈) and a very long polyunsaturated acyl chain (C_{36:5}, C_{40:6}) called a 'phleate' (Fig. 10A). Strikingly, the same molecules have been previously identified as an intermediate of the trehalose polyphleate (TPP) biosynthesis pathway in *M. smegmatis* (Burbaud, Laval et al. 2016). In the latter survey however, they accumulated as a consequence of TPP production failure in a mutant strain depleted for pE acyltranferase involved in TPP metabolism (Fig. 10B-10C). In contrast, *M. smegmatis ΔMSMEG_0948* synthesizes TPP (Fig. 10B-10C), showing that the accumulation of 2-fatty acyl,3-phleate trehalose is likely a secondary effect of the disruption of the envelope lipid content.

### MSMEG_0948 counterparts in other NTM

The HadD protein and coding nucleic acid have the sequences of SEQ ID NO: 1 and 17, respectively, in M. smegmatis.

The HadD protein and coding nucleic acid were identified in NTM other than *M. smegmatis, e.g.,* in *M. avium, M. marinum, M. ulcerans, M. kansasii, M. abscessus, M. chelonae* and M. *fortuitum.*

**Table 1: NTM HadD**

| NTM | SEQ ID NO: | |
|---|---|---|
| | protein | coding nucleic acid |
| *M. smegmatis* | 1 | 17 |
| *M. avium* | 10 | 26 |
| *M. marinum* | 11 | 27 |
| *M. ulcerans* | 12 | 28 |
| *M. kansasii* | 13 | 29 |
| *M. abscessus* | 14 | 30 |
| *M. chelonae* | 15 | 31 |
| *M. fortuitum* | 16 | 32 |

### DISCUSSION

A combination of eGFP-based pull-down, proteomics and bioinformatics analyses led us to discover of a novel partner protein of the mycobacterial FAS-II system, MSMEG_0948 or HadD, in the NTM *M. smegmatis.* HadD is the closest homologue of HadA and HadC proteins, of which it most likely adopts the single hot dog fold. HadA and HadC are the substrate-binding subunits of HadAB and HadBC dehydratases of the FAS-II system, while HadB represents their catalytic subunit (Sacco, Covarrubias et al. 2007; Biswas, Dutta et al. 2015). Interestingly, HadD holds a well conserved catalytic dyad within a degenerate version of the catalytic hydratase 2 motif found in HadB and absent from HadA and HadC, strongly suggesting it is a (*R*)-specific hydratase/dehydratase. Most importantly, HadD is ubiquitous among mycobacteria but, unlike most FAS-II enzymes and particularly HadAB dehydratase, has no ortholog in the mycolic acid-producing related genera. Thus, this protein appears specific to the mycobacterial cells, including *M. leprae* genome that retains a minimal set of genes for cell-wall biosynthesis (Vissa and Brennan 2001). This suggests that its function is not linked to the construction of the common meromycolic medium chain backbone realized by most *Corynebacteriales* FAS-II systems but is rather dedicated to a reaction step specifically required for biosynthesis of the mycobacterial MAs. This is also the case of HadC protein that, associated to HadB, is involved in the late FAS-II elongation steps generating the longest meromycolic chains in mycobacteria. Yet, the MA profile observed for *M. smegmatis hadD* mutant is completely different from that of *M. smegmatis hadC* mutant that still produces the three MA types, α, α' and epoxy, though in proportions different from that found in the wt strain (Jamet, Slama et al. 2015). Furthermore, several physiological parameters, such as the planktonic growth rate, sliding motility, surface hydrophobicity and sensitivity to temperature, SDS and the TB drug ethambutol, are differently affected in both mutants. These data altogether clearly show that HadD function is distinct from that of HadC in *M. smegmatis.*

The inactivation of *hadD* in *M. smegmatis* abolishes the production of both α- and epoxy-MAs. This phenomenon as well as common structural features between these two MA types and the chronological sequence of their formation in wt bacteria (Lacave, Laneelle et al. 1987) strongly support the existence of a direct biosynthetic filiation between these two MA types. Thus, HadD function is dedicated to the synthesis of these long chain MAs. Given that HadD most likely belongs to the (*R*)-specific hydratases/dehydratases family, we propose that it would be responsible for the introduction, using a dehydratation-isomerization mechanism, of the second (proximal) *cis*-double bond into the meromycolic chain. This reaction step would be required for the subsequent formation of the third hydrocarbon segment present in the epoxy- and α-MAs and absent in the α'-MAs (Fig. 6; Fig. 11). Therefore, blocking this step upstream of the α/epoxy-MA biosynthetic split would completely stop their production. Dehydratase-isomerases display a global 3D structure and a catalytic machinery similar to those of simple dehydratases, but exhibit subtle differences in the shape of the substrate-binding tunnel which determine the isomerase potential of the enzyme (Lu, White et al. 2005). This enzyme family uses an anaerobic catalytic mechanism (Leesong, Henderson et al. 1996) distinct from the molecular oxygen-dependent mechanism used by the desaturases (Nagai and Bloch 1968) such as *M. smegmatis* DesA1 desaturase that may be involved in the introduction of one double bond in the α-MAs, as previously proposed (Singh, Varela et al. 2016). The opportunistic pathogen *Pseudomonas aeruginosa* possesses two distinct pathways for unsaturated fatty acid biosynthesis. Like in *Escherichia coli,* the main one uses an anaerobic mechanism dependent on the dehydratase-isomerase FabA belonging to its FAS-II system. The second one is an aerobic inducible pathway governed by the desaturases DesA and DesB (Zhu, Choi et al. 2006). Thus, it is plausible that introduction of double bonds into MAs is also governed by two different mechanisms that would be required to adapt to changes of environmental conditions and specially of oxygen levels. The demonstration of the precise functions of HadD and DesA1 will necessitate enzymatic *in vitro* enzymatic studies.

Profound physiological changes were observed in the *hadD* deficient mutant, where the major component of the mycomembrane correspond to the medium-size α'-MA alone instead of a combination of the three MA types. The NTM *M. smegmatis* can survive *in vitro* with this sole MA. Yet, its planktonic growth is considerably slowed down. Furthermore, its capacities to aggregate or to assemble into colonies or biofilms and to spread by sliding motility are altered, likely due to an increased hydrophobicity of its cell surface reflecting an envelope architecture upheaval, but also to a change in MA content, which plays an important role in biofilm growth (Ojha, Baughn et al. 2008). Interestingly, inactivation of *hadD* also affects, probably indirectly, the production of two families of glycolipids, the TPPs and GPLs, whose biosyntheses are orchestrated by two adjacent clusters on *M. smegmatis* chromosome (Burbaud, Laval et al. 2016). There is a close correlation between the mutant strain features and the marked reduction observed in the content of polar (triglycosylated) GPLs, which are surface-exposed lipids, therefore playing an important role in the cell surface properties, and condition the sliding motility, aggregation and Congo red absorption of *M. smegmatis* (Deshayes, Laval et al. 2005). Moreover, the modification of the envelope composition in MA-containing compounds in the mutant affects the integrity of the bacterial envelope, causing an increased sensitivity to low temperature, SDS and a wide range of antibiotics. The loss of the full-size MAs, *i.e.* epoxy- and alpha-MAs, must contribute to this phenomenon. Indeed, the length of the mycoloyl chains is a major determinant of the fluidity and permeability of mycobacterial cell wall, shorter molecules being associated with higher fluidity (Liu, Barry et al. 1996). The intrinsic resistance of NTM to most conventional antibiotherapies and long-term treatment regimens of moderate efficacy are two main factors of the mortality observed for NTM infection cases (Soni, De Groote et al. 2016). Thus, there is an urgent need for novel therapeutic strategies for the treatment of NTM diseases. The loss of capacities of the mycobacterial *hadD* mutant to multiply, assemble and colonize different kinds of surfaces as well as its hypersensitivity to different kinds of drug, including firstline anti-mycobacterial compounds such as rifampicin, isoniazid and pyrazinamide, makes HadD protein an interesting target for future drug development. Using a combination of molecules including a HadD inhibitor could also represent a relevant approach to improve the antiobiotherapy against pathogenic mycobacteria.

### EXAMPLE 2: HadD M. smegmatis (purification)

Production and purification of HadD. MSMEG_0948 (hadD) gene from *M. smegmatis* mc2155 was cloned between the *Nde*I and *BamH*1 restriction sites of the pET-15b vector (NOVAGEN). The resulting construction was then checked by sequencing. The newly produced plasmid was used to transform chemically competent *E. coli rosetta* (DE3) strain. Bacteria of *E. coli*/pET-15b::*h-hadD* were grown at 37°C in 50 ml LB-medium supplemented with carbenicillin (50 µg/ml) and chloramphenicol (30 µg/ml) until an OD₆₀₀ of 0.9. The temperature was dropped from 37°C to 13°C for 30 min, then 0.2 mM IPTG was added and bacteria further cultured at 13°C for 24 h. After centrifugation, harvested cells were resuspended in 4 ml buffer [50 mM MES (pH 6.5), 500 mM NaCl, 0.5% Triton X-100, 0.5 mg/ml lysozyme, 1 mM AEBSF] and frozen at -80°C overnight. After thawing, 5 µg/ml DNAse, 10 µg/ml RNAse and 10 mM MgCl₂ were added to the lyzed cells. After centrifugation at 15,000 rpm, the total soluble proteins were added to 200 µl of preequilibrated cobalt TALON ® Superflow Metal Affinity resin and shaken for 20 min. Three washing cycles were done using 50 mM MES buffer pH 6.5 supplemented with 500 mM NaCl and 10 mM imidazole and six elution cycles of the resin-bound protein was done using 50 mM MES buffer pH 6.5 supplemented with 500 mM NaCl and 200 mM imidazole. Each purification fraction was analyzed by SDS-PAGE followed by InstantBlue staining (EUROMEDEX).

### EXAMPLE 3: M. smegmatis ΔhadD on macrophages

*M. smegmatis* is knocked out for *hadD* expression, as described in example 1 above. *M. smegmatis ΔhadD* is placed into contact with human alveolar macrophages to determine the capacity *of M. smegmatis ΔhadD* to infect the macrophages, and to compare it with wild-type *M. smegmatis.*

### EXAMPLE 4: HadD in tuberculous mycobacteria

HadD was identified in mycobacteria other than NTM, *e.g.,* in mycobacteria of the *M. tuberculosis* complex (*i.e., M. tuberculosis, M. bovis, M. africanum, M. microti, M. caprae, M. orygis, M. mungi and M. pinnipedii*).

**Table 2: HadD in mycobacteria other than NTM**

| | | | SEQ ID NO: | |
|---|---|---|---|---|
| | | | protein | coding nucleic acid |
| Mycobacteria of the *M. tuberculosis* complex | *M. tuberculosis* | Rv0504c (H37Rv; CDC1551) | 2 | 18 |
| | | ERDMAN_0553 | 3 | 19 |
| | | MRA_0512 | 4 | 20 |
| | *M. bovis* | Mb0516c | 5 | 21 |
| | *M. africanum* | MAF_05110 | 6 | 22 |
| | *M. microti* | | 7 | 23 |
| | *M. canettii* | | 8 | 24 |
| *M. leprae* | | ML2425 | 9 | 25 |

**EXAMPLE 5:** *M. tuberculosis* Δ*hadD* on macrophages; *M. tuberculosis ΔhadD* in mice *M. tuberculosis* is knocked out for *hadD* expression (following the methodology described in examples 1 and 3 above).

*M. tuberculosis ΔhadD* is placed into contact with marcrophages, e.g., human alveolar macrophages, to determine the capacity of *M. tuberculosis ΔhadD* to infect the macrophages, and to compare it with wild-type *M. tuberculosis.*

*M. tuberculosis ΔhadD* is administered to mice via intraveneous injection (or aerosol inhalation), to determine the capacity of *M. tuberculosis ΔhadD* to infect the mice, and to compare it with wild-type *M. tuberculosis.*

### BIBLIOGRAPHIC REFERENCES

Altschul, S. F., T. L. Madden, et al. (1997). "Gapped BLAST and PSI-BLAST: a new generation of protein database search programs." Nucleic Acids Res. 25: 3389-3402.
Biswas, R., A. Dutta, et al. (2015). "Crystal structure of dehydratase component HadAB complex of mycobacterial FAS-II pathway." Biochemical and Biophysical Research Communications 458(2): 369-374.
Burbaud, S., F. Laval, et al. (2016). "Trehalose Polyphleates Are Produced by a Glycolipid Biosynthetic Pathway Conserved across Phylogenetically Distant Mycobacteria." Cell chemical biology 23(2): 278-289.
Cangelosi, G. A., C. O. Palermo, et al. (1999). "Colony morphotypes on Congo red agar segregate along species and drug susceptibility lines in the Mycobacterium avium-intracellulare complex." Microbiology 145 (Pt 6): 1317-1324.
Deshayes, C., F. Laval, et al. (2005). "A glycosyltransferase involved in biosynthesis of triglycosylated glycopeptidolipids in Mycobacterium smegmatis: impact on surface properties." Journal of Bacteriology 187(21): 7283-7291.
Dong, Y., X. Qiu, et al. (2015). "Molecular basis for the inhibition of beta-hydroxyacyl-ACP dehydratase HadAB complex from Mycobacterium tuberculosis by flavonoid inhibitors." Protein & cell 6(7): 504-517.
Etienne, G., C. Villeneuve, et al. (2002). "The impact of the absence of glycopeptidolipids on the ultrastructure, cell surface and cell wall properties, and phagocytosis of Mycobacterium smegmatis." Microbiology 148(Pt 10): 3089-3100.
Hisano, T., T. Tsuge, et al. (2003). "Crystal structure of the (R)-specific enoyl-CoA hydratase from Aeromonas caviae involved in polyhydroxyalkanoate biosynthesis." J. Biol. Chem. 278(1): 617-624.
Jamet, S., Y. Quentin, et al. (2015). "Evolution of Mycolic Acid Biosynthesis Genes and Their Regulation during Starvation in Mycobacterium tuberculosis." Journal of Bacteriology 197(24): 3797-3811.
Jamet, S., N. Slama, et al. (2015). "The Non-Essential Mycolic Acid Biosynthesis Genes hadA and hadC Contribute to the Physiology and Fitness of Mycobacterium smegmatis." PLoS One 10(12): e0145883.
Kapopoulou, A., J. M. Lew, et al. (2011). "The MycoBrowser portal: a comprehensive and manually annotated resource for mycobacterial genomes." Tuberculosis (Edinb) 91(1): 8-13.
Kulka, K., G. Hatfull, et al. (2012). "Growth of Mycobacterium tuberculosis biofilms." J Vis Exp(60).
Laneelle, M. A., A. Launay, et al. (2012). "A novel mycolic acid species defines two novel genera of the Actinobacteria, Hoyosella and Amycolicicoccus." Microbiology 158(Pt 3): 843-855.
Lederer, E. (1969). "Some problems concerning biological C-alkylation reactions and phytosterol biosynthesis." Quarterly Reviews, Chemical Society 23(4): 453-481.
Marrakchi, H., M. A. Laneelle, et al. (2014). "Mycolic acids: structures, biosynthesis, and beyond." Chem Biol 21(1): 67-85.
Martinez, A., S. Torello, et al. (1999). "Sliding motility in mycobacteria." Journal of Bacteriology 181(23): 7331-7338.
Nishiuchi, Y., T. Iwamoto, et al. (2017). "Infection Sources of a Common Non-tuberculous Mycobacterial Pathogen, Mycobacterium avium Complex." Front Med (Lausanne) 4: 27.
North, E. J., M. Jackson, et al. (2014). "New Approaches to Target the Mycolic Acid Biosynthesis Pathway for the Development of Tuberculosis Therapeutics." Curr Pharm Des 20(27): 4357-4378.
Ojha, A. K., A. D. Baughn, et al. (2008). "Growth of Mycobacterium tuberculosis biofilms containing free mycolic acids and harbouring drug-tolerant bacteria." Mol Microbiol 69(1): 164-174.
Ojha, A. K., X. Trivelli, et al. (2010). "Enzymatic hydrolysis of trehalose dimycolate releases free mycolic acids during mycobacterial growth in biofilms." J Biol Chem 285(23): 17380-17389.
Parish, T., Stoker, N. G. (2000). "Use of a flexible cassette method to generate a double unmarked Mycobacterium tuberculosis tlyA plcABC mutant by gene replacement." Microbiology 146: 1969-1975.
Plocinski, P., D. Laubitz, et al. (2014). "Identification of protein partners in mycobacteria using a single-step affinity purification method." PLoS ONE 9(3): e91380.
Pons, J. L. and G. Labesse (2009). "@TOME-2: a new pipeline for comparative modeling of protein-ligand complexes." Nucleic Acids Research 37(Web Server issue): W485-491.
Quemard, A. (2016). "New Insights into the Mycolate-Containing Compound Biosynthesis and Transport in Mycobacteria." Trends in Microbiology 24(9): 725-738.
Sacco, E., A. S. Covarrubias, et al. (2007). "The missing piece of the type II fatty acid synthase system from Mycobacterium tuberculosis." Proc Natl Acad Sci U S A 104(37): 14628-14633.
Sali, A. and T. L. Blundell (1993). "Comparative protein modelling by satisfaction of spatial restraints." J. Mol. Biol. 234: 779-815.
Sievers, F., A. Wilm, et al. (2011). "Fast, scalable generation of high-quality protein multiple sequence alignments using Clustal Omega." Mol Syst Biol 7: 539.
Slama, N., S. Jamet, et al. (2016). "The changes in mycolic acid structures caused by hadC mutation have a dramatic effect on the virulence of Mycobacterium tuberculosis." Molecular Microbiology 99(4): 794-807.
Soni, I., M. A. De Groote, et al. (2016). "Challenges facing the drug discovery pipeline for non-tuberculous mycobacteria." Journal of Medical Microbiology 65(1): 1-8.
Sousa, S., M. Bandeira, et al. (2015). "Nontuberculous mycobacteria pathogenesis and biofilm assembly." Int J Mycobacteriol 4(1): 36-43.
van Kessel, J. C. and G. F. Hatfull (2007). "Recombineering in Mycobacterium tuberculosis." Nat Methods 4(2): 147-152.
Verschoor, J. A., M. S. Baird, et al. (2012). "Towards understanding the functional diversity of cell wall mycolic acids of Mycobacterium tuberculosis." Prog Lipid Res 51(4): 325-339.
Vissa, V. D. and P. J. Brennan (2001). "The genome of Mycobacterium leprae: a minimal mycobacterial gene set." Genome Biol 2(8): REVIEWS1023.
Williams, K. J., G. Joyce, et al. (2010). "Improved mycobacterial tetracycline inducible vectors." Plasmid 64(2): 69-73.

## Claims

1. A protein, the amino acid sequence of which is
- the sequence of SEQ ID NO: 1, or
- an amino acid sequence, which is at least 59% identical to SEQ ID NO: 1 and which has retained the amino acid motif of SEQ ID NO: 39, more particularly an amino acid motif chosen from among SEQ ID NOs: 40-47.

2. The protein of claim 1, which has retained the enzymatic activity of a dehydratase, more particularly the enzymatic activity of a dehydratase and the enzymatic activity of an isomerase.

3. The protein of claim 1 or 2, which is obtainable by isolation (or purification) from a mycobacterium, more particularly from
- a Non-Tuberculous Mycobacterium (NTM), such as *Mycobacterium smegmatis, M. avium, M. marinum, M. ulcerans, M. kansasii, M. abscessus, M. chelonae, M. fortuitum, M. xenopi, M. simiae* and *M. szulgai*; or from
- a non-NTM mycobacterium, such as a mycobacterium of the *M. tuberculosis* complex (M. *tuberculosis, M. bovis, M. africanum, M. microti, M. canettii, M. caprae, M. orygis, M. mungi and M. pinnipedii*)*,* or such as *M. leprae.*

4. The protein of any one of claims 1-3, which is obtainable by
- lysing mycobacterial cells under non-denaturing conditions,
- contacting said lysed mycobacterial cells, or a protein extract thereof, with an affinity ligand or with an antibody, which is covalently linked or binds to at least one bait protein and which co-precipitates proteins that are bound to said at least one bait protein, wherein said at least one bait protein is selected from the group consisting of the Fatty Acid Synthase type II (FAS-II) enzymes of mycobacterial cells, and
- isolating or purifying the protein from the proteins, which co-precipitate with said at least one bait protein.

5. The protein of any one of claims 1-4, which is not obtainable by isolation or purification from *Corynebacterium, Nocardia, Rhodococcus* and *Gordonia.*

6. The protein of any one of claims 1-5, the amino acid sequence of which is chosen from among SEQ ID NO: 1 and 10-16, more particularly from among SEQ ID NO: 1 and 10-13, or
from among SEQ ID NO: 2-9, more particularly from among SEQ ID NO: 2-8.

7. A ligand, which is
- an antibody, or
- a monoclonal antibody, or
- a Fab, Fab' or F(ab)2 fragment, or
- a scFv, or
- a single-domain antibody (sdAb), or
- the variable domain of a sdAb,
wherein said ligand specifically binds the protein of any one of claims 1-6.

8. A method to identify antimycobacterial compound(s), wherein said method comprises
- testing candidate compound(s) for binding to the protein of any one of claims 1-6, and
- selecting the compound(s) that binds(bind) to said protein, more particularly compound(s) that specifically binds(bind) to said protein, wherein selecting said compound(s) identifies it(them) as antimycobacterial compound(s).

9. The method of claim 8, wherein said selected compound(s) are compound(s), which inhibits (inhibit) a dehydratase activity of said protein or polypeptide, or a dehydratase and isomerase activity of said protein or polypeptide.

10. The method of claim 8 or 9, wherein said selected compound(s) are compound(s), which when placed into contact with a mycobacterium, induces(induce) at least one of the following effects:
- the effect of altering the integrity of the envelop of said Mycobacterium;
- the effect of inhibiting the Fatty Acid Synthase type II (FAS-II) of said Mycobacterium;
- the effect of inhibiting the production of alpha-mycolic acids by said *Mycobacterium;*
- the effect of inhibiting the production by said *Mycobacterium* of oxygenated mycolic acid(s), such as epoxy-, methoxy- or keto-mycolic acid(s);
- the effect of inhibiting the capacity or propensity of the cells of said *Mycobacterium* to aggregate or sediment, more particularly when said cells are placed in a liquid culture medium;
- the effect of inducing a change of the morphology of the colony formed by the cells of said *Mycobacterium,* e.g., a change from a rough to a smooth phenotype, more particularly when said cells are placed on a solid culture medium;
- the effect of inhibiting the capacity or propensity of the cells of said *Mycobacterium* to assemble into a biofilm;
- the effect of altering the sliding motility of the cells of said *Mycobacterium;*
- the effect of increasing the sensitivity of said Mycobacterium to a temperature stress, e.g., to a temperature lower than 35°C, e.g., a temperature of 30°C;
- the effect of increasing the sensitivity of said *Mycobacterium* to a denaturing detergent, such as SDS;
- the effect of increasing the sensitivity of said *Mycobacterium* to the human immune system;
- the effect of decreasing the fitness of said *Mycobacterium;*
- the effect of decreasing the resistance of said *Mycobacterium* to an antimycobacterial compound or drug, more particularly to an anti-tuberculosis compound or drug, more particularly to at least one anti-tuberculosis compound or drug chosen from among rifampicin, pyrazinamide, isoxyl, thiacetazone, ethionamide and isoniazid; and
- the effect of increasing the sensitivity of said *Mycobacterium* to an antimycobacterial compound or drug, more particularly to an anti-tuberculosis compound or drug, more particularly to at least one anti-tuberculosis compound or drug chosen from among rifampicin, pyrazinamide, isoxyl, thiacetazone, ethionamide and isoniazid.

11. A nucleic acid, which codes for the protein of any one of claims 1-6.

12. A genetically engineered cell, which recombinantly comprises the nucleic acid of claim 11.

13. A pharmaceutical composition, which comprises the ligand of claim 7, and which optionally further comprises at least one other compound, wherein said at least one other compound is chosen from among the anti-mycobacterial compounds and the anti-tuberculosis compounds.

14. The ligand of claim 7, or the pharmaceutical composition of claim 13, for use in the treatment of a mycobacterial disease.

15. The ligand of claim 7, or the pharmaceutical composition of claim 13, for the use of claim 14, wherein said mycobacterial diseases is a Non-Tuberculous Mycobacterium (NTM) infection, a nosocomial infection, the (myco)bacterial infection of an immunocompromise patient, a non-NTM infection, tuberculosis or leprosy, more particularly a NTM infection, a nosocomial infection or the (myco)bacterial infection of an immunocompromise patient.
